# EUROPEAN PATENT SPECIFICATION

(11) **EP 1 402 065 B1**
(45) Date of publication and mention of the grant of the patent: **28.01.2015**
(21) Application number: 02734638.6
(22) Date of filing: 30.05.2002
(51) Int. Cl.: C12Q 1/68, G01N 33/53, C12P 19/34, G01N 33/68

(54) **METHOD FOR DETECTING TRANSCRIPTION FACTOR-PROTEIN INTERACTIONS**
VERFAHREN ZUM NACHWEIS VON TRANSKRIPTIONSFAKTOR-PROTEIN-WECHSELWIRKUNGEN
METHODE DE DETECTION D'INTERACTIONS ENTRE UNE PROTEINE ET UN FACTEUR DE TRANSCRIPTION

(30) Priority: 08.06.2001 US 877738; 08.06.2001 US 877243; 08.06.2001 US 877403; 08.06.2001 US 877705; 05.09.2001 US 947274
(43) Date of publication of application: 31.03.2004
(73) Proprietor: Affymetrix, Inc., Santa Clara, CA 95051 (US)
(72) Inventor: LI, Xianqiang, Palo Alto, CA 94303 (US)
(74) Representative: Boult Wade Tennant
(86) International application number: PCT/US2002/017408
(87) International publication number: WO 2002/101351

(56) References cited:
- WO-A-02/38734
- WO-A-99/19510
- WO-A-02/052037
- US-A- 5 747 253
- US-A- 5 861 246
- US-A- 6 066 452
- US-A- 6 066 452
- SHUM C H ET AL: "cDNA microarray analysis of Ewing's sarcoma EWS/Fli-1 transcription factor target genes" PROCEEDINGS OF THE AMERICAN ASSOCIATION FOR CANCER RESEARCH ANNUAL MEETING, no. 41, March 2000 (2000-03), page 626, XP001207419 & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X
- BOSS V ET AL: "NERVE GROWTH FACTOR, BUT NOT EPIDERMAL GROWTH FACTOR, INCREASES FRA-2 EXPRESSION AND ALTERS FRA-2/JUND BINDING TO AP-1 AND CREB BINDING ELEMENTS IN PHEOCHROMOCYTOMA (PC12) CELLS" JOURNAL OF NEUROSCIENCE, NEW YORK, NY, US, vol. 21, no. 1, 1 January 2001 (2001-01-01), pages 18-26, XP002909908 ISSN: 0270-6474
- NALLUR G N ET AL: "MULTIPLEX SELECTION TECHNIQUE (MUST): AN APPROACH TO CLONE TRANSCRIPTION FACTOR BINDING SITES" PROCEEDINGS OF THE NATIONAL ACADEMY OF SCIENCES OF USA, NATIONAL ACADEMY OF SCIENCE. WASHINGTON, US, vol. 93, February 1996 (1996-02), pages 1184-1189, XP002921331 ISSN: 0027-8424
- SKOV S ET AL: "Activation of Stat-3 is involved in the induction of apoptosis after ligation of major histocompatibility complex class I molecules on human Jurkat T cells." BLOOD. 15 MAY 1998, vol. 91, no. 10, 15 May 1998 (1998-05-15), pages 3566-3573, XP001207178 ISSN: 0006-4971
- LAM ROBERT ET AL: "An array-based method for specifically profiling multiple transcription factor activity" AMERICAN BIOTECHNOLOGY LABORATORY, vol. 20, no. 8, July 2002 (2002-07), page 22, 26, XP009052228 ISSN: 0749-3223
- KLEIN J. ET AL.: 'A new family of DNA binding proteins includes putative transcriptional regulators of the antirrhinum majus floral meristem identity genes SQUAMOSA' MOLECULAR AND GENERAL GENETICS vol. 250, no. 1, 15 January 1996, pages 7 - 16, XP002961672

### SEQUENCE LISTING

<110> Li, Xianqiang
<120> METHOD FOR DETECTING TRANSCRIPTION FACTOR-PROTEIN
   INTERACTIONS
<130> 26757-705
<150> US 09/877,738
   <151> 2000-06-08
<150> US 09/877,243
   <151> 2000-06-08
<150> US 09/877,403
   <151> 2000-06-08
<150> US 09/877,705
   <151> 2000-06-08
<160> 162
<170> PatentIn version 3.1
<210> 1
   <211> 21
   <212> DNA
   <213> Artificial sequence
<220>
   <223> Transcription factor probe PP01
<400> 1
   cgcttgatga ctcagccgga a
   21
<210> 2
   <211> 21
   <212> DNA
   <213> Artificial sequence
<220>
   <223> Transcription factor probe PP02
<400> 2
   ttccggctga gtcatcaagc g
   21
<210> 3
   <211> 26
   <212> DNA
   <213> Artificial sequence
<220>
   <223> Transcription factor probe PP03
<400> 3
   gatcgaactg accgcccgcg gcccgt
   26
<210> 4
   <211> 26
   <212> DNA
   <213> Artificial sequence
<220>
   <223> Transcription factor probe PP04
<400> 4
   acgggccgcg ggcggtcagt tcgatc
   26
<210> 5
   <211> 23
   <212> DNA
   <213> Artificial sequence
<220>
   <223> Transcription factor probe PP05
<400> 5
   gtctggtaca gggtgttctt ttt
   23
<210> 6
   <211> 23
   <212> DNA
   <213> Artificial sequence
<220>
   <223> Transcription factor probe PP06
<400> 6
   aaaaagaaca ccctgtacca gac
   23
<210> 7
   <211> 18
   <212> DNA
   <213> Artificial sequence
<220>
   <223> Transcription factor probe PP07
<400> 7
   cacagctcat taacgcgc
   18
<210> 8
   <211> 18
   <212> DNA
   <213> Artificial sequence
<220>
   <223> Transcription factor probe PP08
<400> 8
   gcgcgttaat gagctgtg
   18
<210> 9
   <211> 20
   <212> DNA
   <213> Artificial sequence
<220>
   <223> Transcription factor probe PP09
<400> 9
   tgcagattgc gcaatctgca
   20
<210> 10
   <211> 20
   <212> DNA
   <213> Artificial sequence
<220>
   <223> Transcription factor probe PP10
<400> 10
   tgcagattgc gcaatctgca
   20
<210> 11
   <211> 27
   <212> DNA
   <213> Artificial sequence
<220>
   <223> Transcription factor probe PP11
<400> 11
   agaccgtacg tgattggtta atctctt
   27
<210> 12
   <211> 27
   <212> DNA
   <213> Artificial sequence
<220>
   <223> Transcription factor probe PP12
<400> 12
   aagagattaa ccaatcacgt acggtct
   27
<210> 13
   <211> 27
   <212> DNA
   <213> Artificial sequence
<220>
   <223> Transcription factor probe PP13
<400> 13
   acccaatgat tattagccaa tttctga
   27
<210> 14
   <211> 27
   <212> DNA
   <213> Artificial sequence
<220>
   <223> Transcription factor probe PP14
<400> 14
   tcagaaattg gctaataatc attgggt
   27
<210> 15
   <211> 25
   <212> DNA
   <213> Artificial sequence
<220>
   <223> Transcription factor probe PP15
<400> 15
   tacaggcata acggttccgt agtga
   25
<210> 16
   <211> 25
   <212> DNA
   <213> Artificial sequence
<220>
   <223> Transcription factor probe PP16
<400> 16
   tcactacgga accgttatgc ctgta
   25
<210> 17
   <211> 27
   <212> DNA
   <213> Artificial sequence
<220>
   <223> Transcription factor probe PP17
<400> 17
   agagattgcc tgacgtcaga gagctag
   27
<210> 18
   <211> 27
   <212> DNA
   <213> Artificial sequence
<220>
   <223> Transcription factor probe PP18
<400> 18
   ctagctctct gacgtcaggc aatctct
   27
<210> 19
   <211> 25
   <212> DNA
   <213> Artificial sequence
<220>
   <223> Transcription factor probe PP19
<400> 19
   atttaagttt cgcgcccttt ctcaa
   25
<210> 20
   <211> 25
   <212> DNA
   <213> Artificial sequence
<220>
   <223> Transcription factor probe PP20
<400> 20
   ttgagaaagg gcgcgaaact taaat
   25
<210> 21
   <211> 27
   <212> DNA
   <213> Artificial sequence
<220>
   <223> Transcription factor probe PP21
<400> 21
   ggatccagcg ggggcgagcg ggggcca
   27
<210> 22
   <211> 27
   <212> DNA
   <213> Artificial sequence
<220>
   <223> Transcription factor probe PP22
<400> 22
   tggcccccgc tcgcccccgc tggatcc
   27
<210> 23
   <211> 35
   <212> DNA
   <213> Artificial sequence
<220>
   <223> Transcription factor probe PP23
<400> 23
   gtccaaagtc aggtcacagt gacctgatca aagtt
   35
<210> 24
   <211> 35
   <212> DNA
   <213> Artificial sequence
<220>
   <223> Transcription factor probe PP24
<400> 24
   aactttgatc aggtcactgt gacctgactt tggac
   35
<210> 25
   <211> 31
   <212> DNA
   <213> Artificial sequence
<220>
   <223> Transcription factor probe PP25
<400> 25
   ggaggagggc tgcttgagga agtataagaa t
   31
<210> 26
   <211> 31
   <212> DNA
   <213> Artificial sequence
<220>
   <223> Transcription factor probe PP26
<400> 26
   attcttatac ttcctcaagc agccctcctc c
   31
<210> 27
   <211> 21
   <212> DNA
   <213> Artificial sequence
<220>
   <223> Transcription factor probe PP27
<400> 27
   gatctcgagc aggaagttcg a
   21
<210> 28
   <211> 21
   <212> DNA
   <213> Artificial sequence
<220>
   <223> Transcription factor probe PP28
<400> 28
   tcgaacttcc tgctcgagat c
   21
<210> 29
   <211> 21
   <212> DNA
   <213> Artificial sequence
<220>
   <223> Transcription factor probe PP29
<400> 29
   cggattgtgt attggctgta c
   21
<210> 30
   <211> 21
   <212> DNA
   <213> Artificial sequence
<220>
   <223> Transcription factor probe PP30
<400> 30
   gtacagccaa tacacaatcc g
   21
<210> 31
   <211> 32
   <212> DNA
   <213> Artificial sequence
<220>
   <223> Transcription factor probe PP31
<400> 31
   cgaagtactt tcagtttcat attactctac aa
   32
<210> 32
   <211> 32
   <212> DNA
   <213> Artificial sequence
<220>
   <223> Transcription factor probe PP32
<400> 32
   ttgtagagta atatgaaact gaaagtactt cg
   32
<210> 33
   <211> 27
   <212> DNA
   <213> Artificial sequence
<220>
   <223> Transcription factor probe PP33
<400> 33
   cacttgataa cagaaagtga taactct
   27
<210> 34
   <211> 27
   <212> DNA
   <213> Artificial sequence
<220>
   <223> Transcription factor probe PP34
<400> 34
   agagttatca ctttctgtta tcaagtg
   27
<210> 35
   <211> 41
   <212> DNA
   <213> Artificial sequence
<220>
   <223> Transcription factor probe PP35
<400> 35
   gaccctagag gatctgtaca ggatgttcta gatccaattc g
   41
<210> 36
   <211> 41
   <212> DNA
   <213> Artificial sequence
<220>
   <223> Transcription factor probe PP36
<400> 36
   cgaattggat ctagaacatc ctgtacagat cctctagggt c
   41
<210> 37
   <211> 25
   <212> DNA
   <213> Artificial sequence
<220>
   <223> Transcription factor probe PP37
<400> 37
   ctcagcttgt actttggtac aacta
   25
<210> 38
   <211> 25
   <212> DNA
   <213> Artificial sequence
<220>
   <223> Transcription factor probe PP38
<400> 38
   tagttgtacc aaagtacaag ctgag
   25
<210> 39
   <211> 22
   <212> DNA
   <213> Artificial sequence
<220>
   <223> Transcription factor probe PP39
<400> 39
   ggaagcgaaa atgaaattga ct
   22
<210> 40
   <211> 22
   <212> DNA
   <213> Artificial sequence
<220>
   <223> Transcription factor probe PP40
<400> 40
   agtcaatttc attttcgctt cc
   22
<210> 41
   <211> 25
   <212> DNA
   <213> Artificial sequence
<220>
   <223> Transcription factor probe PP41
<400> 41
   gatcccccca acacctgctg cctga
   25
<210> 42
   <211> 25
   <212> DNA
   <213> Artificial sequence
<220>
   <223> Transcription factor probe PP42
<400> 42
   tcaggcagca ggtgttgggg ggatc
   25
<210> 43
   <211> 25
   <212> DNA
   <213> Artificial sequence
<220>
   <223> Transcription factor probe PP43
<400> 43
   gatcgctcta aaaataaccc tgtcg
   25
<210> 44
   <211> 25
   <212> DNA
   <213> Artificial sequence
<220>
   <223> Transcription factor probe PP44
<400> 44
   cgacagggtt atttttagag cgatc
   25
<210> 45
   <211> 26
   <212> DNA
   <213> Artificial sequence
<220>
   <223> Transcription factor probe PP45
<400> 45
   ggaagcagac cacgtggtct gcttcc
   26
<210> 46
   <211> 26
   <212> DNA
   <213> Artificial sequence
<220>
   <223> Transcription factor probe PP46
<400> 46
   ggaagcagac cacgtggtct gcttcc
   26
<210> 47
   <211> 25
   <212> DNA
   <213> Artificial sequence
<220>
   <223> Transcription factor probe PP47
<400> 47
   ttttggattg aagccaatat gataa
   25
<210> 48
   <211> 25
   <212> DNA
   <213> Artificial sequence
<220>
   <223> Transcription factor probe PP48
<400> 48
   ttatcatatt ggcttcaatc caaaa
   25
<210> 49
   <211> 30
   <212> DNA
   <213> Artificial sequence
<220>
   <223> Transcription factor probe PP49
<400> 49
   acgcccaaag aggaaaattt gtttcataca
   30
<210> 50
   <211> 30
   <212> DNA
   <213> Artificial sequence
<220>
   <223> Transcription factor probe PP50
<400> 50
   tgtatgaaac aaattttcct ctttgggcgt
   30
<210> 51
   <211> 27
   <212> DNA
   <213> Artificial sequence
<220>
   <223> Transcription factor probe PP51
<400> 51
   cgctccgcgg ccatcttggc ggctggt
   27
<210> 52
   <211> 27
   <212> DNA
   <213> Artificial sequence
<220>
   <223> Transcription factor probe PP52
<400> 52
   accagccgcc aagatggccg cggagcg
   27
<210> 53
   <211> 27
   <212> DNA
   <213> Artificial sequence
<220>
   <223> Transcription factor probe PP53
<400> 53
   tggggaacct gtgctgagtc actggag
   27
<210> 54
   <211> 27
   <212> DNA
   <213> Artificial sequence
<220>
   <223> Transcription factor probe PP54
<400> 54
   ctccagtgac tcagcacagg ttcccca
   27
<210> 55
   <211> 22
   <212> DNA
   <213> Artificial sequence
<220>
   <223> Transcription factor probe PP55
<400> 55
   agttgagggg actttcccag gc
   22
<210> 56
   <211> 22
   <212> DNA
   <213> Artificial sequence
<220>
   <223> Transcription factor probe PP56
<400> 56
   gcctgggaaa gtcccctcaa ct
   22
<210> 57
   <211> 22
   <212> DNA
   <213> Artificial sequence
<220>
   <223> Transcription factor probe PP57
<400> 57
   tgtcgaatgc aaatcactag aa
   22
<210> 58
   <211> 22
   <212> DNA
   <213> Artificial sequence
<220>
   <223> Transcription factor probe PP58
<400> 58
   ttctagtgat ttccattcga ca
   22
<210> 59
   <211> 27
   <212> DNA
   <213> Artificial sequence
<220>
   <223> Transcription factor probe PP59
<400> 59
   tacagaacat gtctaagcat gctgggg
   27
<210> 60
   <211> 27
   <212> DNA
   <213> Artificial sequence
<220>
   <223> Transcription factor probe PP60
<400> 60
   ccccagcatg cttagacatg ttctgta
   27
<210> 61
   <211> 27
   <212> DNA
   <213> Artificial sequence
<220>
   <223> Transcription factor probe PP61
<400> 61
   gaatggggca ctgaggcgtg accaccg
   27
<210> 62
   <211> 27
   <212> DNA
   <213> Artificial sequence
<220>
   <223> Transcription factor probe PP62
<400> 62
   cggtggtcac gcctcagtgc cccattc
   27
<210> 63
   <211> 26
   <212> DNA
   <213> Artificial sequence
<220>
   <223> Transcription factor probe PP63
<400> 63
   cgaattgatt gatgcactaa ttggag
   26
<210> 64
   <211> 26
   <212> DNA
   <213> Artificial sequence
<220>
   <223> Transcription factor probe PP64
<400> 64
   ctccaattag tgcatcaatc aattcg
   26
<210> 65
   <211> 28
   <212> DNA
   <213> Artificial sequence
<220>
   <223> Transcription factor probe PP65
<400> 65
   tgtcttcctg aatatgaata agaaataa
   28
<210> 66
   <211> 28
   <212> DNA
   <213> Artificial sequence
<220>
   <223> Transcription factor probe PP66
<400> 66
   ttatttctta ttcatattca ggaagaca
   28
<210> 67
   <211> 20
   <212> DNA
   <213> Artificial sequence
<220>
   <223> Transcription factor probe PP67
<400> 67
   caaaactagg tcaaaggtca
   20
<210> 68
   <211> 20
   <212> DNA
   <213> Artificial sequence
<220>
   <223> Transcription factor probe PP68
<400> 68
   tgacctttga cctagttttg
   20
<210> 69
   <211> 27
   <212> DNA
   <213> Artificial sequence
<220>
   <223> Transcription factor probe PP69
<400> 69
   gatcctgtac aggatgttct agctaca
   27
<210> 70
   <211> 27
   <212> DNA
   <213> Artificial sequence
<220>
   <223> Transcription factor probe PP70
<400> 70
   tgtagctaga acatcctgta caggatc
   27
<210> 71
   <211> 31
   <212> DNA
   <213> Artificial sequence
<220>
   <223> Transcription factor probe PP71
<400> 71
   tcgagggtag ggttcaccga aagttcactc g
   31
<210> 72
   <211> 31
   <212> DNA
   <213> Artificial sequence
<220>
   <223> Transcription factor probe PP72
<400> 72
   cgagtgaact ttcggtgaac cctaccctcg a
   31
<210> 73
   <211> 26
   <212> DNA
   <213> Artificial sequence
<220>
   <223> Transcription factor probe PP73
<400> 73
   agcttcaggt cagaggtcag agagct
   26
<210> 74
   <211> 26
   <212> DNA
   <213> Artificial sequence
<220>
   <223> Transcription factor probe PP74
<400> 74
   agctctctga cctctgacct gaagct
   26
<210> 75
   <211> 27
   <212> DNA
   <213> Artificial sequence
<220>
   <223> Transcription factor probe PP75
<400> 75
   gtgcatttcc cgtaaatctt gtctaca
   27
<210> 76
   <211> 27
   <212> DNA
   <213> Artificial sequence
<220>
   <223> Transcription factor probe PP76
<400> 76
   tgtagacaag atttacggga aatgcac
   27
<210> 77
   <211> 16
   <212> DNA
   <213> Artificial sequence
<220>
   <223> Transcription factor probe PP77
<400> 77
   agtatgtcta gactga
   16
<210> 78
   <211> 16
   <212> DNA
   <213> Artificial sequence
<220>
   <223> Transcription factor probe PP78
<400> 78
   tcagtctaga catact
   16
<210> 79
   <211> 39
   <212> DNA
   <213> Artificial sequence
<220>
   <223> Transcription factor probe PP79
<400> 79
   tcgagagcca gacaaaaagc cagacattta gccagacac
   39
<210> 80
   <211> 39
   <212> DNA
   <213> Artificial sequence
<220>
   <223> Transcription factor probe PP80
<400> 80
   gtgtctggct aaatgtctgg ctttttgtct ggctctcga
   39
<210> 81
   <211> 21
   <212> DNA
   <213> Artificial sequence
<220>
   <223> Transcription factor probe PP81
<400> 81
   attcgatcgg ggcggggcga g
   21
<210> 82
   <211> 21
   <212> DNA
   <213> Artificial sequence
<220>
   <223> Transcription factor probe PP82
<400> 82
   ctcgccccgc cccgatcgaa t
   21
<210> 83
   <211> 22
   <212> DNA
   <213> Artificial sequence
<220>
   <223> Transcription factor probe PP83
<400> 83
   ggatgtccat attaggacat ct
   22
<210> 84
   <211> 22
   <212> DNA
   <213> Artificial sequence
<220>
   <223> Transcription factor probe PP84
<400> 84
   agatgtccta atatggacat cc
   22
<210> 85
   <211> 25
   <212> DNA
   <213> Artificial sequence
<220>
   <223> Transcription factor probe PP85
<400> 85
   catgttatgc atattcctgt aagtg
   25
<210> 86
   <211> 25
   <212> DNA
   <213> Artificial sequence
<220>
   <223> Transcription factor probe PP86
<400> 86
   cacttacagg aatatgcata acatg
   25
<210> 87
   <211> 24
   <212> DNA
   <213> Artificial sequence
<220>
   <223> Transcription factor probe PP87
<400> 87
   gatccttctg ggaattccta gatc
   24
<210> 88
   <211> 24
   <212> DNA
   <213> Artificial sequence
<220>
   <223> Transcription factor probe PP88
<400> 88
   gatctaggaa ttcccagaag gatc
   24
<210> 89
   <211> 33
   <212> DNA
   <213> Artificial sequence
<220>
   <223> Transcription factor probe PP89
<400> 89
   ctagagcctg atttccccga aatgatgagc tag
   33
<210> 90
   <211> 33
   <212> DNA
   <213> Artificial sequence
<220>
   <223> Transcription factor probe PP90
<400> 90
   ctagctcatc atttcgggga aatcaggctc tag
   33
<210> 91
   <211> 21
   <212> DNA
   <213> Artificial sequence
<220>
   <223> Transcription factor probe PP91
<400> 91
   agatttctag gaattcaatc c
   21
<210> 92
   <211> 21
   <212> DNA
   <213> Artificial sequence
<220>
   <223> Transcription factor probe PP92
<400> 92
   ggattgaatt cctagaaatc t
   21
<210> 93
   <211> 20
   <212> DNA
   <213> Artificial sequence
<220>
   <223> Transcription factor probe PP93
<400> 93
   gtatttccca gaaaaggaac
   20
<210> 94
   <211> 20
   <212> DNA
   <213> Artificial sequence
<220>
   <223> Transcription factor probe PP94
<400> 94
   gttccttttc tgggaaatac
   20
<210> 95
   <211> 25
   <212> DNA
   <213> Artificial sequence
<220>
   <223> Transcription factor probe PP95
<400> 95
   gcagagcata taaaatgagg tagga
   25
<210> 96
   <211> 25
   <212> DNA
   <213> Artificial sequence
<220>
   <223> Transcription factor probe PP96
<400> 96
   tcctacctca ttttatatgc tctgc
   25
<210> 97
   <211> 32
   <212> DNA
   <213> Artificial sequence
<220>
   <223> Transcription factor probe PP97
<400> 97
   gatcgtaaga ttcaggtcat gacctgagga ga
   32
<210> 98
   <211> 32
   <212> DNA
   <213> Artificial sequence
<220>
   <223> Transcription factor probe PP98
<400> 98
   tctcctcagg tcatgacctg aatcttacga tc
   32
<210> 99
   <211> 29
   <212> DNA
   <213> Artificial sequence
<220>
   <223> Transcription factor probe PP99
<400> 99
   agcttcaggt cacaggaggt cagagagct
   29
<210> 100
   <211> 29
   <212> DNA
   <213> Artificial sequence
<220>
   <223> Transcription factor probe PP100
<400> 100
   agctctctga cctcctgtga cctgaagct
   29
<210> 101
   <211> 23
   <212> DNA
   <213> Artificial sequence
<220>
   <223> Transcription factor probe PP101
<400> 101
   cacccggtca cgtggcctac acc
   23
<210> 102
   <211> 23
   <212> DNA
   <213> Artificial sequence
<220>
   <223> Transcription factor probe PP102
<400> 102
   ggtgtaggcc acgtgaccgg gtg
   23
<210> 103
   <211> 28
   <212> DNA
   <213> Artificial sequence
<220>
   <223> Transcription factor probe PP103
<400> 103
   agcttcaggt caaggaggtc agagagct
   28
<210> 104
   <211> 28
   <212> DNA
   <213> Artificial sequence
<220>
   <223> Transcription factor probe PP104
<400> 104
   agctctctga cctccttgac ctgaagct
   28
<210> 105
   <211> 15
   <212> DNA
   <213> Artificial sequence
<220>
   <223> Transcription factor probe PP105
<400> 105
   ctggaatttt ctaga
   15
<210> 106
   <211> 15
   <212> DNA
   <213> Artificial sequence
<220>
   <223> Transcription factor probe PP106
<400> 106
   tctagaaaat tccag
   15
<210> 107
   <211> 15
   <212> DNA
   <213> Artificial sequence
<220>
   <223> Transcription factor probe PP107
<400> 107
   ctctgcgccc ggccc
   15
<210> 108
   <211> 15
   <212> DNA
   <213> Artificial sequence
<220>
   <223> Transcription factor probe PP108
<400> 108
   gggccgggcg cagag
   15
<210> 109
   <211> 63
   <212> DNA
   <213> Artificial sequence
<220>
   <223> Hybridization probe MP02
<400> 109
   ttccggctga gtcatcaagc gttccggctg agtcatcaag cgttccggct gagtcatcaa 60
   gcg
   63
<210> 110
   <211> 78
   <212> DNA
   <213> Artificial sequence
<220>
   <223> Hybridization probe MP04
<400> 110
   acgggccgcg ggcggtcagt tcgatcacgg gccgcgggcg gtcagttcga tcacgggccg 60
   cgggcggtca gttcgatc
   78
<210> 111
   <211> 69
   <212> DNA
   <213> Artificial sequence
<220>
   <223> Hybridization probe MP06-1
<400> 111
   aaaaagaaca ccctgtacca gacaaaaaga acaccctgta ccagacaaaa agaacaccct 60
   gtaccagac
   69
<210> 112
   <211> 54
   <212> DNA
   <213> Artificial sequence
<220>
   <223> Hybridization probe MP08
<400> 112
<210> 113
   <211> 60
   <212> DNA
   <213> Artificial sequence
<220>
   <223> Hybridization probe MP10
<400> 113
<210> 114
   <211> 81
   <212> DNA
   <213> Artificial sequence
<220>
   <223> Hybridization probe MP12
<400> 114
<210> 115
   <211> 81
   <212> DNA
   <213> Artificial sequence
<220>
   <223> Hybridization probe MP14
<400> 115
<210> 116
   <211> 75
   <212> DNA
   <213> Artificial sequence
<220>
   <223> Hybridization probe MP16
<400> 116
   tcactacgga accgttatgc ctgtatcact acggaaccgt tatgcctgta tcactacgga 60
   accgttatgc ctgta 75
<210> 117
   <211> 81
   <212> DNA
   <213> Artificial sequence
<220>
   <223> Hybridization probe MP18
<400> 117
   ctagctctct gacgtcaggc aatctctcta gctctctgac gtcaggcaat ctctctagct 60
   ctctgacgtc aggcaatctc t 81
<210> 118
   <211> 75
   <212> DNA
   <213> Artificial sequence
<220>
   <223> Hybridization probe MP20
<400> 118
   ttgagaaagg gcgcgaaact taaatttgag aaagggcgcg aaacttaaat ttgagaaagg 60
   gcgcgaaact taaat 75
<210> 119
   <211> 81
   <212> DNA
   <213> Artificial sequence
<220>
   <223> Hybridization probe MP22
<400> 119
   tggcccccgc tcgcccccgc tggatcctgg cccccgctcg cccccgctgg atcctggccc 60
   ccgctcgccc ccgctggatc c 81
<210> 120
   <211> 70
   <212> DNA
   <213> Artificial sequence
<220>
   <223> Hybridization probe MP24
<400> 120
   aactttgatc aggtcactgt gacctgactt tggacaactt tgatcaggtc actgtgacct 60
   gactttggac 70
<210> 121
   <211> 93
   <212> DNA
   <213> Artificial sequence
<220>
   <223> Hybridization probe MP26
<400> 121
   attcttatac ttcctcaagc agccctcctc cattcttata cttcctcaag cagccctcct 60
   ccattcttat acttcctcaa gcagccctcc tcc 93
<210> 122
   <211> 63
   <212> DNA
   <213> Artificial sequence
<220>
   <223> Hybridization probe MP28
<400> 122
<210> 123
   <211> 63
   <212> DNA
   <213> Artificial sequence
<220>
   <223> Hybridization probe MP30
<400> 123
<210> 124
   <211> 96
   <212> DNA
   <213> Artificial sequence
<220>
   <223> Hybridization probe MP32
<400> 124
<210> 125
   <211> 81
   <212> DNA
   <213> Artificial sequence
<220>
   <223> Hybridization probe MP34
<400> 125
<210> 126
   <211> 82
   <212> DNA
   <213> Artificial sequence
<220>
   <223> Hybridization probe MP36
<400> 126
   cgaattggat ctagaacatc ctgtacagat cctctagggt ccgaattgga tctagaacat 60
   cctgtacaga tcctctaggg tc 82
<210> 127
   <211> 75
   <212> DNA
   <213> Artificial sequence
<220>
   <223> Hybridization probe MP38
<400> 127
   tagttgtacc aaagtacaag ctgagtagtt gtaccaaagt acaagctgag tagttgtacc 60
   aaagtacaag ctgag 75
<210> 128
   <211> 66
   <212> DNA
   <213> Artificial sequence
<220>
   <223> Hybridization probe MP40
<400> 128
   agtcaatttc attttcgctt ccagtcaatt tcattttcgc ttccagtcaa tttcattttc 60
   gcttcc 66
<210> 129
   <211> 75
   <212> DNA
   <213> Artificial sequence
<220>
   <223> Hybridization probe MP42
<400> 129
<210> 130
   <211> 75
   <212> DNA
   <213> Artificial sequence
<220>
   <223> Hybridization probe MP44
<400> 130
<210> 131
   <211> 78
   <212> DNA
   <213> Artificial sequence
<220>
   <223> Hybridization probe MP46
<400> 131
<210> 132
   <211> 75
   <212> DNA
   <213> Artificial sequence
<220>
   <223> Hybridization probe MP48
<400> 132
   ttatcatatt ggcttcaatc caaaattatc atattggctt caatccaaaa ttatcatatt 60
   ggcttcaatc caaaa 75
<210> 133
   <211> 90
   <212> DNA
   <213> Artificial sequence
<220>
   <223> Hybridization probe MP50
<400> 133
   tgtatgaaac aaattttcct ctttgggcgt tgtatgaaac aaattttcct ctttgggcgt 60
   tgtatgaaac aaattttcct ctttgggcgt 90
<210> 134
   <211> 81
   <212> DNA
   <213> Artificial sequence
<220>
   <223> Hybridization probe MP52
<400> 134
   accagccgcc aagatggccg cggagcgacc agccgccaag atggccgcgg agcgaccagc 60
   cgccaagatg gccgcggagc g 81
<210> 135
   <211> 81
   <212> DNA
   <213> Artificial sequence
<220>
   <223> Hybridization probe MP54
<400> 135
   ctccagtgac tcagcacagg ttccccactc cagtgactca gcacaggttc cccactccag 60
   tgactcagca caggttcccc a 81
<210> 136
   <211> 66
   <212> DNA
   <213> Artificial sequence
<220>
   <223> Hybridization probe MP56
<400> 136
<210> 137
   <211> 66
   <212> DNA
   <213> Artificial sequence
<220>
   <223> Hybridization probe MP58
<400> 137
<210> 138
   <211> 81
   <212> DNA
   <213> Artificial sequence
<220>
   <223> Hybridization probe MP60
<400> 138
<210> 139
   <211> 81
   <212> DNA
   <213> Artificial sequence
<220>
   <223> Hybridization probe MP62
<400> 139
   cggtggtcac gcctcagtgc cccattccgg tggtcacgcc tcagtgcccc attccggtgg 60
   tcacgcctca gtgccccatt c 81
<210> 140
   <211> 78
   <212> DNA
   <213> Artificial sequence
<220>
   <223> Hybridization probe MP64
<400> 140
   ctccaattag tgcatcaatc aattcgctcc aattagtgca tcaatcaatt cgctccaatt 60
   agtgcatcaa tcaattcg 78
<210> 141
   <211> 84
   <212> DNA
   <213> Artificial sequence
<220>
   <223> Hybridization probe MP66
<400> 141
   ttatttctta ttcatattca ggaagacatt atttcttatt catattcagg aagacattat 60
   ttcttattca tattcaggaa gaca 84
<210> 142
   <211> 60
   <212> DNA
   <213> Artificial sequence
<220>
   <223> Hybridization probe MP68
<400> 142
   tgacctttga cctagttttg tgacctttga cctagttttg tgacctttga cctagttttg 60
<210> 143
   <211> 81
   <212> DNA
   <213> Artificial sequence
<220>
   <223> Hybridization probe MP70
<400> 143
   tgtagctaga acatcctgta caggatctgt agctagaaca tcctgtacag gatctgtagc 60
   tagaacatcc tgtacaggat c 81
<210> 144
   <211> 93
   <212> DNA
   <213> Artificial sequence
<220>
   <223> Hybridization probe MP72
<400> 144
   cgagtgaact ttcggtgaac cctaccctcg acgagtgaac tttcggtgaa ccctaccctc 60
   gacgagtgaa ctttcggtga accctaccct cga 93
<210> 145
   <211> 78
   <212> DNA
   <213> Artificial sequence
<220>
   <223> Hybridization probe MP74
<400> 145
   agctctctga cctctgacct gaagctagct ctctgacctc tgacctgaag ctagctctct 60
   gacctctgac ctgaagct 78
<210> 146
   <211> 81
   <212> DNA
   <213> Artificial sequence
<220>
   <223> Hybridization probe MP76
<400> 146
   tgtagacaag atttacggga aatgcactgt agacaagatt tacgggaaat gcactgtaga 60
   caagatttac gggaaatgca c 81
<210> 147
   <211> 64
   <212> DNA
   <213> Artificial sequence
<220>
   <223> Hybridization probe MP78
<400> 147
   tcagtctaga catacttcag tctagacata cttcagtcta gacatacttc agtctagaca 60
   tact 64
<210> 148
   <211> 117
   <212> DNA
   <213> Artificial sequence
<220>
   <223> Hybridization probe MP80
<400> 148
   gtgtctggct aaatgtctgg ctttttgtct ggctctcgag tgtctggcta aatgtctggc 60
   tttttgtctg gctctcgagt gtctggctaa atgtctggct ttttgtctgg ctctcga 117
<210> 149
   <211> 63
   <212> DNA
   <213> Artificial sequence
<220>
   <223> Hybridization probe MP82
<400> 149
   ctcgccccgc cccgatcgaa tctcgccccg ccccgatcga atctcgcccc gccccgatcg 60
   aat 63
<210> 150
   <211> 66
   <212> DNA
   <213> Artificial sequence
<220>
   <223> Hybridization probe MP84
<400> 150
   agatgtccta atatggacat ccagatgtcc taatatggac atccagatgt cctaatatgg 60
   acatcc 66
<210> 151
   <211> 75
   <212> DNA
   <213> Artificial sequence
<220>
   <223> Hybridization probe MP86
<400> 151
   cacttacagg aatatgcata acatgcactt acaggaatat gcataacatg cacttacagg 60
   aatatgcata acatg 75
<210> 152
   <211> 72
   <212> DNA
   <213> Artificial sequence
<220>
   <223> Hybridization probe MP88
<400> 152
   gatctaggaa ttcccagaag gatcgatcta ggaattccca gaaggatcga tctaggaatt 60
   cccagaagga tc 72
<210> 153
   <211> 99
   <212> DNA
   <213> Artificial sequence
<220>
   <223> Hybridization probe MP90
<400> 153
<210> 154
   <211> 63
   <212> DNA
   <213> Artificial sequence
<220>
   <223> Hybridization probe MP92
<400> 154
<210> 155
   <211> 60
   <212> DNA
   <213> Artificial sequence
<220>
   <223> Hybridization probe MP94
<400> 155
<210> 156
   <211> 75
   <212> DNA
   <213> Artificial sequence
<220>
   <223> Hybridization probe MP96
<400> 156
<210> 157
   <211> 96
   <212> DNA
   <213> Artificial sequence
<220>
   <223> Hybridization probe MP98
<400> 157
<210> 158
   <211> 87
   <212> DNA
   <213> Artificial sequence
<220>
   <223> Hybridization probe MP100
<400> 158
<210> 159
   <211> 69
   <212> DNA
   <213> Artificial sequence
<220>
   <223> Hybridization probe MP102
<400> 159
<210> 160
   <211> 84
   <212> DNA
   <213> Artificial sequence
<220>
   <223> Hybridization probe MP104
<400> 160
   agctctctga cctccttgac ctgaagctag ctctctgacc tccttgacct gaagctagct 60
   ctctgacctc cttgacctga agct 84
<210> 161
   <211> 60
   <212> DNA
   <213> Artificial sequence
<220>
   <223> Hybridization probe MP106
<400> 161
   tctagaaaat tccagtctag aaaattccag tctagaaaat tccagtctag aaaattccag 60
<210> 162
   <211> 60
   <212> DNA
   <213> Artificial sequence
<220>
   <223> Hybridization probe MP108
<400> 162
   gggccgggcg cagaggggcc gggcgcagag gggccgggcg cagaggggcc gggcgcagag 60

## Description

### Field of the Invention

The present invention relates to methods for detecting activated transcription factors in a cell sample. More specifically, the invention relates to methods for detecting interactions between activated transcription factors in a cell sample and compositions, kits, and uses arising there from.

### Description of Related Art

All living organisms use nucleic acids (DNA and RNA) to encode the genes which make up the genome for that organism. Each gene encodes a protein that may be produced by the organism through expression of the gene.

It is important to note that the mere presence of a gene in a cell does not communicate the functionality of that gene to the cell. Rather, it is only when the gene is expressed and a protein is produced that the functionality of the gene encoding the protein is conveyed.

The systems that regulate gene expression respond to a wide variety of developmental and environmental stimuli, thus allowing each cell type to express a unique and characteristic subset of its genes, and to adjust the dosage of particular gene products as needed. The importance of dosage control is underscored by the fact that targeted disruption of key regulatory molecules in mice often results in drastic phenotypic abnormalities [Johnson, R. S., et al., Cell, 71:577-586 (1992)], just as inherited or acquired defects in the function of genetic regulatory mechanisms contribute broadly to human disease.

The importance of controlled gene expression in human disease and the information available to date relating to the mechanisms of gene regulation have fueled efforts aimed at discovering ways of overriding endogenous regulatory controls or of creating new signaling circuitry in cells [Belshaw, P. J., et al., Proc. Natl. Acad. Sci. USA, 93:4604-4607 (1996); Ho, S. H., et al., Nature (London), 382:822-826 (1996); Rivera, V. M., et al., Nat. Med., 2:1028-1032; Spencer, D. M., et al., Science, 262:1019-1024 (1993)].

Critical to this research are effective tools for monitoring gene expression. It is therefore of interest to be able to rapidly and accurately determine the relative expression of different genes in different cells, tissues and organisms, over time, and under various conditions, treatments and regimes. As will be described herein in greater detail, there are a great many applications that arise from being able to effectively monitor which genes are being expressed by a given cell at a given time.

Standard molecular biology techniques have been used to analyze the expression of genes in a cell by measuring DNA. These techniques include PCR, northern blot analysis, or other types of DNA probe analysis such as *in situ* hybridization. Each of these methods allows one to analyze the transcription of only known genes and/or small numbers of genes at a time. Nucl. Acids Res. 19, 7097-7104 (1991); Nucl. Acids Res. 18,4833-4842 (1990); Nucl. Acids Res. 18, 2789-2792 (1989); European J. Neuroscience 2, 1063-1073 (1990); Analytical Biochem. 187, 364-373 (1990); Genet. Annal Techn. Appl. 7, 64-70 (1990); GATA 8(4), 129-133 (1991); Proc. Natl. Acad. Sci. USA 85, 1696-1700 (1988); Nucl. Acids Res. 19, 1954 (1991); Proc. Natl. Acad. Sci. USA 88, 1943-1947 (1991); Nucl. Acids Res. 19, 6123-6127 (1991); Proc. Natl. Acad. Sci. USA 85, 5738-5742 (1988); Nucl. Acids Res. 16, 10937 (1988).

Gene expression has also been monitored by measuring levels of mRNA. Since proteins are transcribed from mRNA, it is possible to detect transcription by measuring the amount of mRNA present. One common method, called "hybridization subtraction", allows one to look for changes in gene expression by detecting changes in mRNA expression. Nucl. Acids Res. 19, 7097-7104 (1991); Nucl. Acids Res. 18,4833-4842 (1990); Nucl. Acids Res. 18,2789-2792 (1989); European J. Neuroscience 2, 1063-1073 (1990); Analytical Biochem. 187, 364-373 (1990); Genet. Annal Techn. Appl. 7, 64-70 (1990); GATA 8(4), 129-133 (1991); Proc. Natl. Acad. Sci. USA 85,1696-1700 (1988); Nucl. Acids Res. 19, 1954 (1991); Proc. Natl. Acad. Sci. USA 88, 1943-1947 (1991); Nucl. Acids Res. 19, 6123-6127 (1991); Proc. Natl. Acad. Sci. USA 85, 5738-5742 (1988); Nucl. Acids Res. 16, 10937 (1988).

Gene expression has also been monitored by measuring levels of gene product, (i.e., the expressed protein), in a cell, tissue, organ system, or even organism. Measurement of gene expression by measuring the protein gene product may be performed using antibodies known to bind to a particular protein to be detected. A difficulty arises in needing to generate antibodies to each protein to be detected. Measurement of gene expression via protein detection may also be performed using 2-dimensional gel electrophoresis, wherein proteins can be, in principle, identified and quantified as individual bands, and ultimately reduced to a discrete signal. In order to positively analyze each band, each band must be excised from the membrane and subjected to protein sequence analysis using Edman degradation. Unfortunately, it tends to be difficult to isolate a sufficient amount of protein to obtain a reliable sequence. In addition, many of the bands contain more than one discrete protein.

A further difficulty associated with quantifying gene expression by measuring an amount of protein gene product in a cell is that protein expression is an indirect measure of gene expression. It is impossible to known from a protein present in a cell when that protein was expressed by the cell. As a result, it is hard to determine whether protein expression changes over time due to cells being exposed to different stimuli.

Gene expression has also been monitored by measuring the amount of particular activated transcription factors present in a cell. Transcription in a cell is controlled by proteins, referred to herein as "activated transcription factors" which bind to DNA at sites outside the core promoter for the gene and activate transcription. Since activated transcription factors activate transcription, detection of their presence is useful for measuring gene expression. Transcriptional activators are found in prokaryotes, viruses, and eukaryotes, including fungi, plants, and animals, including mammals, providing a wide range of therapeutic targets.

The regulatory mechanisms controlling the transcription of protein-coding genes by RNA polymerase II have been extensively studied. RNA polymerase II and its host of associated proteins are recruited to the core promoter through non-covalent contacts with sequence-specific DNA binding proteins [Tjian, R. and Maniatis, T., Cell, 77:5-8 (1994); Stringer, K. F., Nature (London), 345:783-786 (1990)]. An especially prevalent and important subset of such proteins, known as transcription factors, typically bind DNA at sites outside the core promoter and activate transcription through space contacts with components of the transcriptional machinery, including chromatin remodeling proteins [Tjian, R. and Maniatis, T., Cell, 77:5-8 (1994); Stringer, K. F., Nature (London), 345:783-786 (1990); Bannister, A. J. and Kouzarides, T., Nature, 384:641-643 (1996); Mizzen, C. A., et al., Cell, 87:1261-1270 (1996)]. The DNA-binding and activation functions of transcription factors generally reside on separate domains whose operation is portable to heterologous fusion proteins [Sadowski, I., et al., Nature, 335:563-564 (1988)]. Though it is believed that activation domains are physically associated with a DNA-binding domain to attain proper function, the linkage between the two need not be covalent [Belshaw, P. J., et al., Proc. Natl. Acad. Sci. USA, 93:4604-4607 (1996); Ho, S. H., et al., Nature (London), 382:822-826 (1996)]. In many instances, the activation domain does not appear to contact the transcriptional machinery directly, but rather through the intermediacy of adapter proteins known as coactivators [Silverman, N., et al., Proc. Natl. Acad. Sci. USA, 91:11005-11008 ((1994); Arany, Z., et al., Nature (London), 374:81-84 (1995)].

One of the difficulties associated with measuring gene expression by measuring transcription factors is that one must measure the subset of transcription factors which are "activated." Certain post-transcriptional modifications occur which render transcription factors "active" in the sense that they are capable of binding to DNA. It is thus necessary to distinguish between activated and non-activated transcription factors so that the "activated transcription factors" can be selectively measured.

Several different methods have been developed for detecting activated transcription factors. One method involves using antibodies selective for activated transcription factors over inactive forms of the transcription factor. This method is impractical for detecting multiple different activated transcription factors due to difficulties associated with developing numerous different antibodies having the requisite bind specificities.

Another method for detecting activated transcription factors involves measuring DNA - transcription factor complexes through a gel shift assay. [Ausebel, F.M. et al eds (1993) Current Protocols in Molecular Biology Vol.2 Greene Publishing Associates, Inc. and John Wiley and Sons, Inc., New York]. According to this method, a sample containing an activated transcription factor is contacted with a DNA probe that comprises a recognition sequence for the transcription factor. A complex between the activated transcription factor and the DNA probe is formed. The DNA-protein complex is detected by a gel-shift assay. Since individual gel shift assays must be performed for each activated transcription factor - DNA complex, this method is currently impractical for measuring multiple different activated transcription factors at the same time.

U.S. Patent Nos. 6,066,452 and 5,861,246 describe methods for determining DNA binding sites for DNA-binding proteins. The DNA binding sites may then be used as probes to isolate DNA-binding proteins. Similarly, PCT Publication No. WO 00/04196 describes methods for identifying cis acting nucleic acid elements as well as methods for isolating nucleic acid binding factors. WO 99/19510 describes a synthetic array of double-stranded nucleic acid molecules comprising protein recognition sites.

### SUMMARY OF THE INVENTION

The invention provides a method for identifying which of a plurality of different activated transcription factors are present in a biological sample, the method comprising:
contacting the biological sample with a library of double stranded DNA probes under conditions where DNA probe - transcription factor complexes are formed between the DNA probes in the library and activated transcription factors present in the biological sample, the DNA probes each comprising a known transcription factor recognition sequence varying within the library such that different DNA probes bind different activated transcription factors, wherein a different DNA probe is provided for each different activated transcription factor in the plurality of activated transcription factors;
isolating the DNA probes that have bound to the activated transcription factors and formed the DNA probe - transcription factor complexes; and
contacting the isolated DNA probes with an array of immobilized hybridization probes under conditions suitable for hybridization of at least one of the strands of the different double stranded DNA probes to the hybridization probes in the array, wherein a different hybridization probe is provided for each different double stranded DNA probe, and wherein identification of the DNA probes bound to the array identifies which of the plurality of different activated transcription factors are present in the biological sample.

The present invention relates to methods and kits for isolating DNA probes that bind to activated transcription factors.

One method comprises: contacting a biological sample with a library of double stranded DNA probes under conditions where DNA probe - transcription factor complexes are formed between the DNA probes and activated transcription factors present in the biological sample; separating DNA probe - transcription factor complexes from non-complexed DNA probes in the library using an agarose gel separation; excising a portion of the agarose gel comprising the separated DNA probe - transcription factor complexes; and isolating the DNA probes from the excised portion of the agarose gel.

Another method comprises: contacting a biological sample with a library of double stranded DNA probes under conditions where DNA probe - transcription factor complexes are formed between the DNA probes and activated transcription factors present in the biological sample; separating DNA probe - transcription factor complexes from non-complexed DNA probes in the library using an agarose gel separation; excising a portion of the agarose gel comprising the separated DNA probe - transcription factor complexes; isolating the DNA probes from the excised portion of the agarose gel; and identifying which of the DNA probes in the library are isolated.

The invention also provides a kit for use in identifying which of a plurality of different activated transcription factors are present in a biological sample by identifying which members of a library of double stranded DNA probes form DNA probe - transcription factor complexes with activated transcription factors present in the biological sample, the kit comprising:
the library of double stranded DNA probes, the DNA probes each comprising a known transcription factor recognition sequence varying within the library such that different DNA probes bind different activated transcription factors, wherein a different DNA probe is provided for each different activated transcription factor in the plurality of activated transcription factors; and
a hybridization array comprising:
   a substrate; and
   a plurality of hybridization probes immobilized on a surface of the substrate such that different hybridization probes are positioned in different defined regions on the surface, wherein a different hybridization probe is provided for each different double stranded DNA probe, and wherein each different hybridization probe comprises a transcription factor probe binding region comprising at least two copies of a complement to a portion of the transcription factor recognition sequence on one of the DNA probes.

A kit is described herein which comprises: a library of double stranded DNA probes, each probe comprising a recognition sequence to which an activated transcription factor is capable of binding and forming a DNA probe - transcription factor complex, the DNA probes in the library capable of forming DNA probe - transcription factor complexes with multiple different activated transcription factors; and instructions for separating DNA probe-transcription factor complexes from non-complexed DNA probes in the library by agarose gel separation.

Kits are also described herein for DNA probe libraries for detecting activated transcription factors.

In one instance, the kit comprises: first and second libraries of double stranded DNA probes, each probe in the first and second libraries comprising a recognition sequence to which an activated transcription factor is capable of binding and forming a DNA probe-transcription factor complex, the DNA probes in the library capable of forming DNA probe-transcription factor complexes with multiple different activated transcription factors; wherein the probes of the first library further comprise a first detectable marker and the probes of the second library further comprise a second detectable marker that is different than the first detectable marker.

Methods, arrays and kits are also provided for detecting activated transcription factors using a hybridization array.

In one instance, a method is provided which comprises: taking a library of double stranded transcription factor probes, the transcription factor probes each comprising a recognition sequence capable of binding to an activated transcription factor, the recognition sequence varying within the library for binding to different activated transcription factors; contacting a biological sample with the library of double stranded DNA probes under conditions where DNA probe - transcription factor complexes are formed between the DNA probes and activated transcription factors present in the biological sample; isolating the transcription factor probes from the transcription factor probe - transcription factor complexes formed; and identifying which transcription factor probes in the library formed complexes by taking an array of immobilized hybridization probes capable of hybridizing to at least one of the strands of the different double stranded transcription factor probes in the library and contacting the isolated transcription factor probes with the array under conditions suitable for hybridization of the strands of the different double stranded transcription factor probes to the hybridization probes in the array.

In another instance, a hybridization array is provided for use in identifying which of a plurality of different activated transcription factors are present in a biological sample by immobilizing transcription factor probes that form transcription factor probe - transcription factor complexes with different activated transcription factors, the array comprising: a substrate; and a plurality of hybridization probes immobilized on a surface of the substrate such that different hybridization probes are positioned in different defined regions on the surface, the different hybridization probes comprising a different transcription factor probe binding region capable of immobilizing a different transcription factor probe to the array, the transcription factor probe binding region comprising at least two copies of a compliment to a portion of a recognition sequence comprised on the transcription factor probe. The hybridization array may optionally further comprise an internal standard. For example, the array may further comprise biotinylated DNA which is employed as an internal standard.

In another instance, a kit is described for use in identifying which of a plurality of different activated transcription factors are present in a biological sample by isolating and immobilizing transcription factor probes that form transcription factor probe - transcription factor complexes with different activated transcription factors, the kit comprising: a hybridization array comprising a substrate, and a plurality of hybridization probes immobilized on a surface of the substrate such that different hybridization probes are positioned in different defined regions on the surface, the different hybridization probes comprising a different transcription factor probe binding region capable of immobilizing a different transcription factor probe to the array, the transcription factor probe binding region comprising at least two copies of a compliment to a portion of a recognition sequence comprised on the transcription factor probe; and instructions for separating DNA probe-transcription factor complexes from non-complexed DNA probes in the library by agarose gel separation.

Methods, arrays and kits are also described herein for identifying complexes between transcription factors and other proteins including other transcription factors. It is noted that these methods, arrays and kits may be applied more broadly for detecting complexes between proteins.

One method comprises identifying complexes between a transcription factor and another protein, the method comprising: isolating from a biological sample transcription factor complexes based on whether the transcription factor complexes comprise a particular type of transcription factor; and identifying which of a plurality of different proteins are present in the isolated transcription factor complexes.

According to this method, identifying which of a plurality of different proteins are present in the isolated transcription factor complexes may comprise identifying which of a plurality of different transcription factors are present in the isolated transcription factor complexes. Identifying which of a plurality of different proteins are present in the isolated transcription factor complexes may also comprise identifying which of a plurality of different non-transcription factor proteins are present in the isolated transcription factor complexes. Identifying which of a plurality of different proteins are present in the isolated transcription factor complexes may also comprise detecting which of a plurality of different transcription factor and non-transcription factor proteins are present in the isolated transcription factor complexes. Identifying which of a plurality of different types of proteins are present in the isolated transcription factor complexes may also comprise simultaneously identifying multiple different types of non-transcription factor proteins in a same assay.

Isolating transcription factor complexes may optionally comprise contacting the biological sample with an agent that selectively binds to the particular type of transcription factor; and isolating those transcription factor complexes to which the agent binds.

Isolating transcription factor complexes may also optionally comprise contacting the biological sample with an agent that selectively binds to the particular type of transcription factor; precipitating those transcription factor complexes to which the agent binds; and isolating the precipitate.

Isolating transcription factor complexes may also optionally comprise contacting the biological sample with an agent immobilized on a solid support that selectively binds to the particular type of transcription factor; and isolating transcription factor complexes immobilized on the solid support through binding to the agent.

Isolating transcription factor complexes may also optionally comprise contacting the biological sample with an antibody that selectively binds to the particular type of transcription factor; and isolating those transcription factor complexes to which the antibody binds.

Identifying which of a plurality of different types of proteins are present in the isolated transcription factor complexes may comprise simultaneously identifying multiple different types of proteins and/or transcription factors in a same assay.

Simultaneously identifying multiple different types of proteins in a same assay may comprise simultaneously identifying at least 5, 10, 15, 20, 50 or more different types of transcription factors in a same assay.

Simultaneously identifying multiple different types of transcription factors in a same assay may optionally be based on a characterization of which different types of transcription factors are present in the isolated transcription factor complexes.

Simultaneously identifying multiple different types of transcription factors in a same assay may also optionally be based on an identification of transcription factor probes isolated from the isolated transcription factor complexes.

When the method comprises identifying transcription factors, the method may optionally further comprise, prior to isolating transcription factor complexes, contacting the biological sample with a library of transcription factor probes under conditions where transcription factor probe - transcription factor complexes are formed between the transcription factor probes and activated transcription factors present in the biological sample; and identifying which of a plurality of different types of transcription factors are present in the isolated transcription factor complexes comprises identifying which transcription factor probes are present in the isolated transcription factor complexes.

When the method comprises identifying transcription factors, the method may also optionally comprise, prior to isolating transcription factor complexes, taking a library of transcription factor probes, the transcription factor probes each comprising a recognition sequence capable of binding to an activated transcription factor, the recognition sequence varying within the library for binding to different activated transcription factors, and contacting the biological sample with the library of transcription factor probes under conditions where transcription factor probe - transcription factor complexes are formed between the transcription factor probes and activated transcription factors present in the biological sample; wherein identifying which of a plurality of different types of transcription factors are present in the isolated transcription factor complexes comprises identifying which transcription factor probes are present in the isolated transcription factor complexes.

When the method comprises identifying transcription factors, the method may optionally comprise, prior to selectively isolating transcription factor complexes, taking a library of transcription factor probes, the transcription factor probes each comprising a recognition sequence capable of binding to an activated transcription factor, the recognition sequence varying within the library for binding to different activated transcription factors, and contacting a biological sample with the library of transcription factor probes under conditions where transcription factor probe - transcription factor complexes are formed between the transcription factor probes and activated transcription factors present in the biological sample; wherein identifying which of a plurality of different types of transcription factors are present in the isolated transcription factor complexes comprises taking an array of immobilized hybridization probes capable of hybridizing to the transcription factor probes in the library and contacting the isolated transcription factor probes with the array under conditions suitable for hybridization of the different transcription factor probes to the hybridization probes in the array.

A method for identifying complexes between a protein and a plurality of transcription factors is described herein which comprises: isolating from a biological sample transcription factor complexes based on whether the transcription factor complexes comprise a particular type of non-transcription factor protein; and identifying which of a plurality of different transcription factors are present in the isolated transcription factor complexes.

According to this method, isolating transcription factor complexes may optionally comprise contacting the biological sample with an agent that selectively binds to the particular type of non-transcription factor protein; and isolating those transcription factor complexes to which the agent binds. Isolating transcription factor complexes may also optionally comprise contacting the biological sample with an agent that selectively binds to the particular type of non-transcription factor protein; precipitating those transcription factor complexes to which the agent binds; and isolating the precipitate. Isolating transcription factor complexes may also optionally comprise contacting the biological sample with an agent immobilized on a solid support that selectively binds to the particular type of non-transcription factor protein; and isolating transcription factor complexes immobilized on the solid support through binding to the agent. Isolating transcription factor complexes may also optionally comprise contacting the biological sample with an antibody that selectively binds to the particular type of non-transcription factor protein; and isolating those transcription factor complexes to which the antibody binds.

Isolating transcription factor complexes may also optionally comprise simultaneously identifying multiple different types of transcription factors in a same assay. Simultaneously identifying multiple different types of transcription factors in a same assay may comprise simultaneously identifying at least 5, 10, 15, 20, 50 or more different types of transcription factors in a same assay.

Methods for characterizing cell types based on which activated transcription factors are present in a sample are also described.

One method comprises: taking a library of double stranded transcription factor probes, the transcription factor probes each comprising a recognition sequence capable of binding to an activated transcription factor, the recognition sequence varying within the library for binding to different activated transcription factors native to different cell types; contacting a biological sample with the library of double stranded DNA probes under conditions where DNA probe - transcription factor complexes are formed between the DNA probes and activated transcription factors present in the biological sample; isolating the transcription factor probes from the transcription factor probe - transcription factor complexes formed; identifying which transcription factor probes in the library formed complexes by taking an array of immobilized hybridization probes capable of hybridizing to at least one of the strands of the different double stranded transcription factor probes in the library and contacting the isolated transcription factor probes with the array under conditions suitable for hybridization of the strands of the different double stranded transcription factor probes to the hybridization probes in the array; and identifying a cell type of the biological sample based on which transcription factor probes are identified.

Methods for identifying a disease state based on which activated transcription factors are present in a biological sample are also described.

One method comprises taking a library of double stranded transcription factor probes, the transcription factor probes each comprising a recognition sequence capable of binding to an activated transcription factor, the recognition sequence varying within the library for binding to different activated transcription factors native to different cell types; identifying which activated transcription factors are present in a nuclear extract of a test sample of cells by: contacting the nuclear extract of the test sample with the library of double stranded DNA probes under conditions where DNA probe - transcription factor complexes are formed between the DNA probes and activated transcription factors present in the test sample, isolating the transcription factor probes from the transcription factor probe - transcription factor complexes formed, and identifying which transcription factor probes in the library formed complexes by taking an array of immobilized hybridization probes capable of hybridizing to at least one of the strands of the different double stranded transcription factor probes in the library and contacting the isolated transcription factor probes with the array under conditions suitable for hybridization of the strands of the different double stranded transcription factor probes to the hybridization probes in the array; identifying which activated transcription factors are present in a nuclear extract of a control sample of cells by: contacting the nuclear extract of the control sample with the library of double stranded DNA probes under conditions where DNA probe - transcription factor complexes are formed between the DNA probes and activated transcription factors present in the control sample, isolating the transcription factor probes from the transcription factor probe - transcription factor complexes formed, and identifying which transcription factor probes in the library formed complexes by taking an array of immobilized hybridization probes capable of hybridizing to at least one of the strands of the different double stranded transcription factor probes in the library and contacting the isolated transcription factor probes with the array under conditions suitable for hybridization of the strands of the different double stranded transcription factor probes to the hybridization probes in the array; and comparing which activated transcription factors are present in the test sample and the control sample.

Methods for screening drug candidates for modulating an activated transcription factor's activity are also described.

One method comprises: forming a plurality of test samples by contacting samples of cells with different agents; and for each test sample, identifying which of a plurality of different activated transcription factors are present by: taking a library of double stranded transcription factor probes, the transcription factor probes each comprising a recognition sequence capable of binding to an activated transcription factor, the recognition sequence varying within the library for binding to different activated transcription factors, contacting the different test sample with the library of double stranded DNA probes under conditions where DNA probe - transcription factor complexes are formed between the DNA probes and activated transcription factors present in the test samples, isolating the transcription factor probes from the transcription factor probe - transcription factor complexes formed, and identifying which transcription factor probes in the library formed complexes by taking an array of immobilized hybridization probes capable of hybridizing to at least one of the strands of the different double stranded transcription factor probes in the library and contacting the isolated transcription factor probes with the array under conditions suitable for hybridization of the strands of the different double stranded transcription factor probes to the hybridization probes in the array; and comparing the activated transcription factors present in the different test samples.

Methods for determining sequence binding requirements for an activated transcription factor are also described.

One method comprises: contacting a sample comprising an activated transcription factor with a library of double stranded DNA probes under conditions where DNA probe-transcription factor complexes are formed between the DNA probes and the activated transcription factor; separating DNA probe - transcription factor complexes from non- complexed DNA probes in the library; isolating the DNA probes from the excised portion of the agarose gel; and determining a consensus sequence for the DNA probes isolated in order to assess the binding requirements for the transcription factor.

Another method comprises contacting a sample comprising an activated transcription factor with a library of double stranded DNA probes under conditions where DNA probe-transcription factor complexes are formed between the DNA probes and the activated transcription factor; separating DNA probe - transcription factor complexes from non-complexed DNA probes in the library using an agarose gel separation; excising a portion of the agarose gel comprising the separated DNA probe - transcription factor complexes; isolating the DNA probes from the excised portion of the agarose gel; and determining a consensus sequence for the DNA probes isolated in order to assess the binding requirements for the transcription factor.

Yet another method comprises: contacting a sample comprising an activated transcription factor with a library of double stranded DNA probes under conditions where DNA probe - transcription factor complexes are formed between the DNA probes and the activated transcription factor;
separating DNA probe - transcription factor complexes from non-complexed DNA probes in the library; isolating the DNA probes from the excised portion of the agarose gel; and quantifying the amount of each of the isolated DNA probes.

Yet another method comprises: contacting a sample comprising an activated transcription factor with a library of double stranded DNA probes under conditions where DNA probe - transcription factor complexes are formed between the DNA probes and the activated transcription factor; separating DNA probe - transcription factor complexes from non-complexed DNA probes in the library using an agarose gel separation; excising a portion of the agarose gel comprising the separated DNA probe - transcription factor complexes; isolating the DNA probes from the excised portion of the agarose gel; and quantifying the amount of each of the isolated DNA probes.

Methods are also described for quantifying expression and activation of multiple different activated transcription factors. One method comprises: contacting a biological sample with a library of double stranded DNA probes for detecting active forms of multiple different transcription factors under conditions where DNA probe - transcription factor complexes are formed between the DNA probes and activated transcription factors present in the biological sample; isolating DNA probes from the DNA probe - transcription factor complexes; identifying which of the multiple different transcription factors are present in an activated form in the biological sample based on which DNA probes are isolated; and quantifying expression of the multiple different transcription factors from cDNA for the biological sample.

### BRIEF DESCRIPTION OF THE DRAWINGS

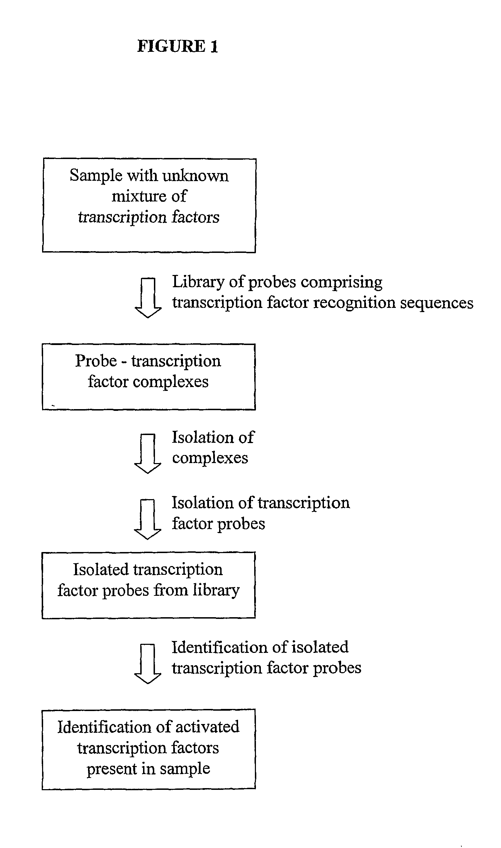

Figure 1 provides a flow diagram for a method for identifying which of a plurality of transcription factors are activated in a given sample of cells.

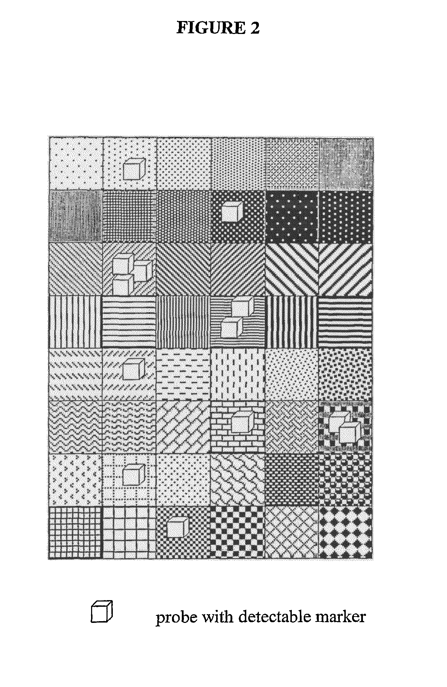

Figure 2 illustrates an array of hybridization probes attached to a solid support where different hybridization probes are attached to discrete, different regions of the array. A transcription factor expression signature is shown based on the distribution of where transcription factor probes hybridize and their intensity.

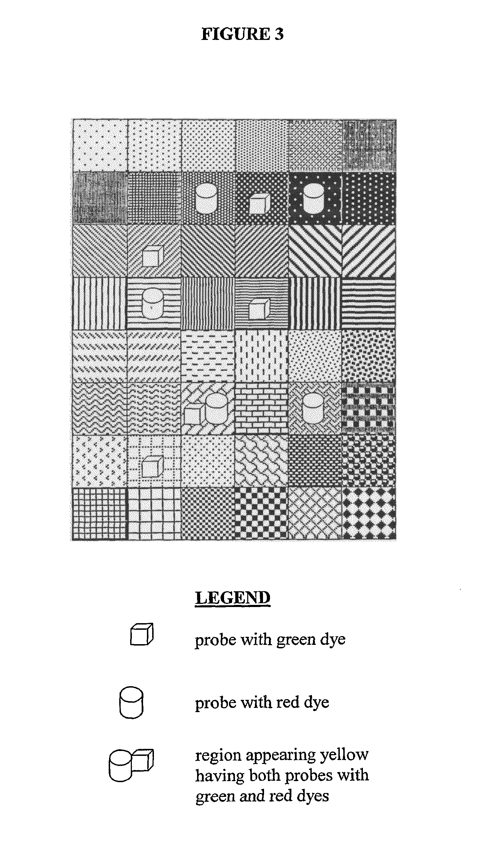

Figure 3 illustrates an array of hybridization probes attached to a solid support where probes from a first sample with a first color dye and probes from a second sample with a second color dye are both contacted with the array.

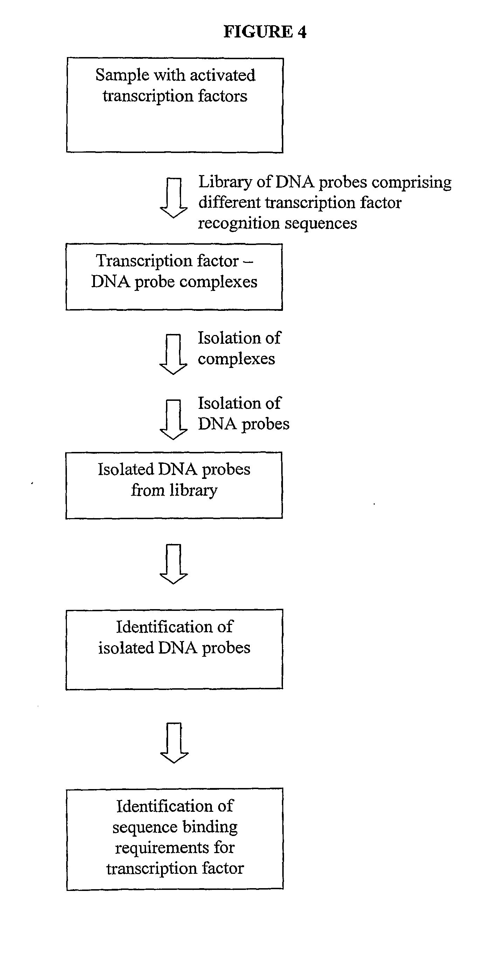

Figure 4 illustrates a process whereby the minimum DNA sequence binding requirements for a given transcription factor can be rapidly determined.

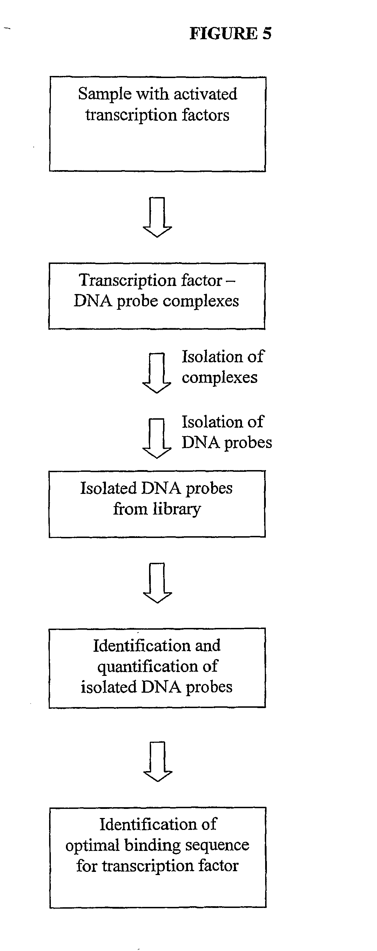

Figure 5 illustrates a variation of the method described in regard to Figure 4 where an optimal sequence for binding is identified.

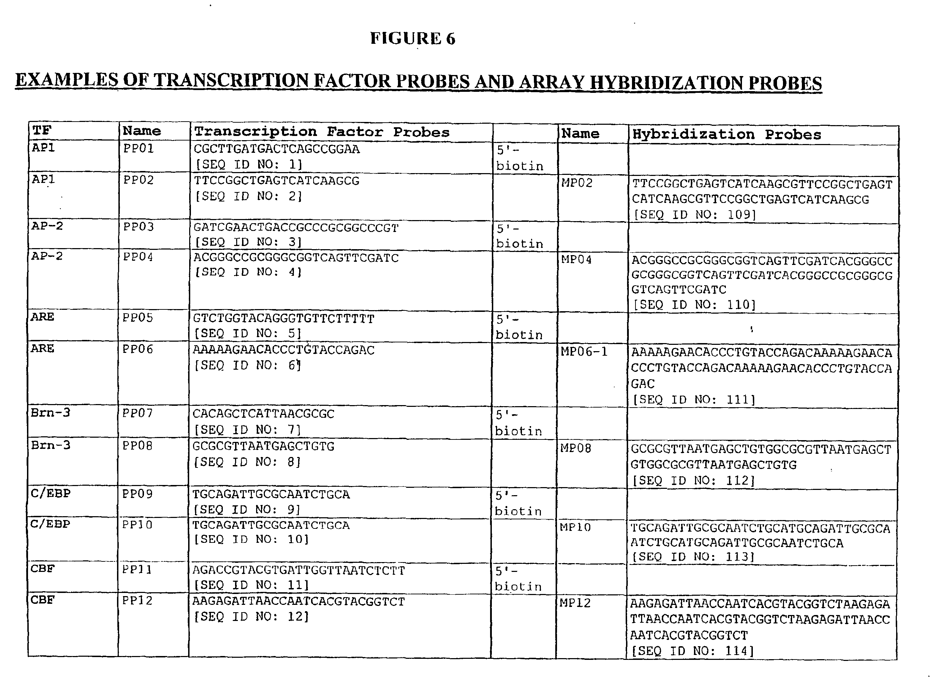

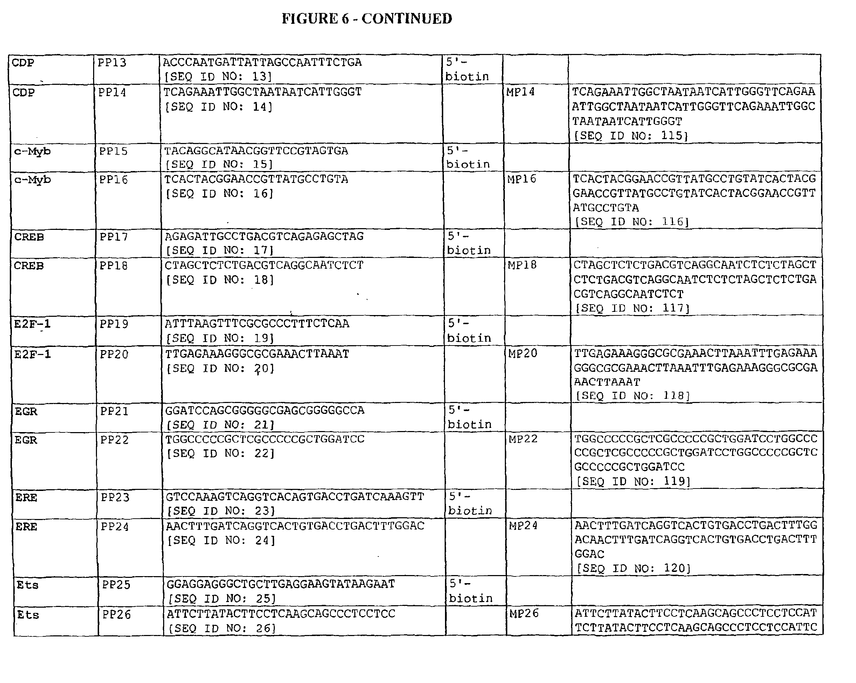

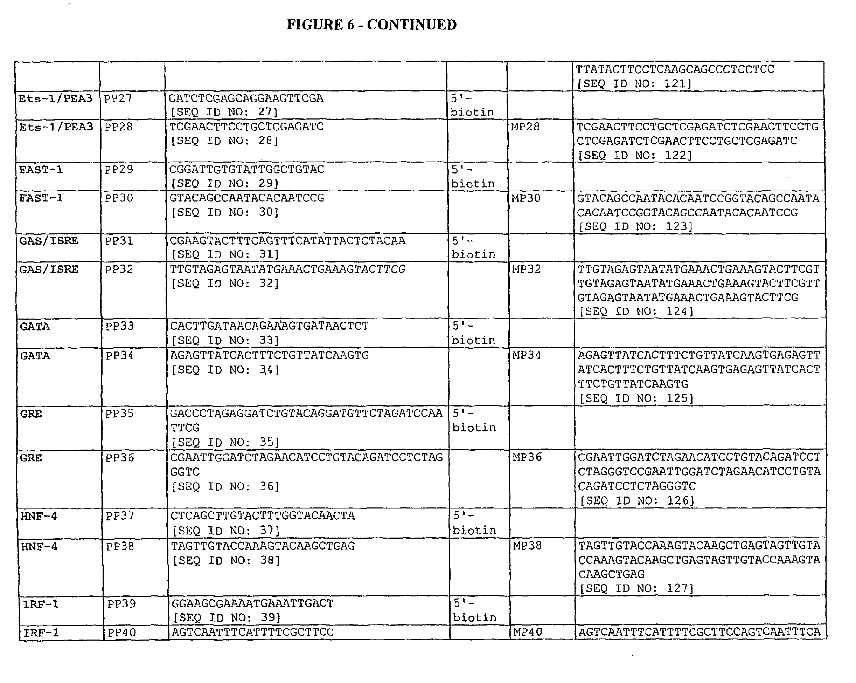

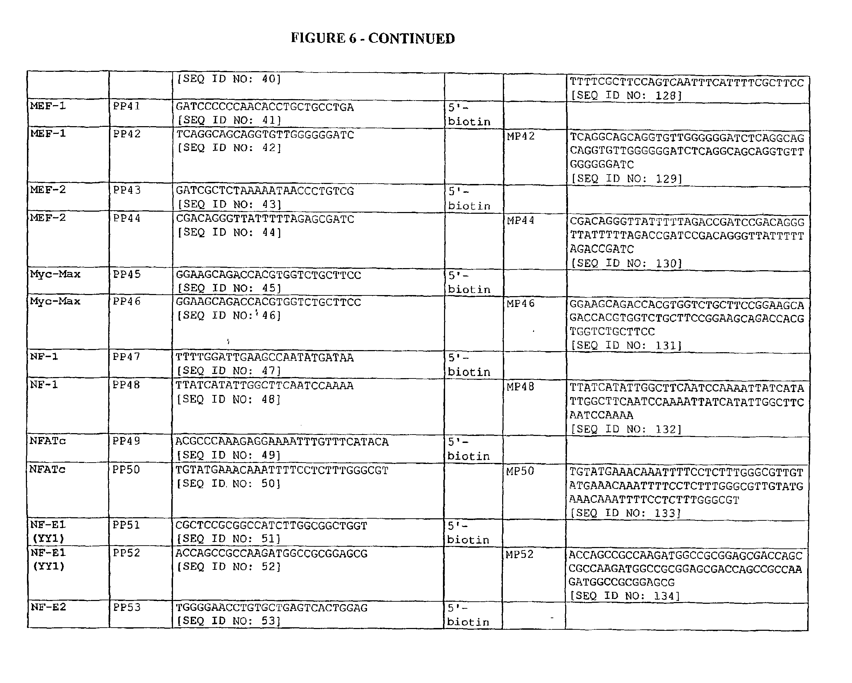

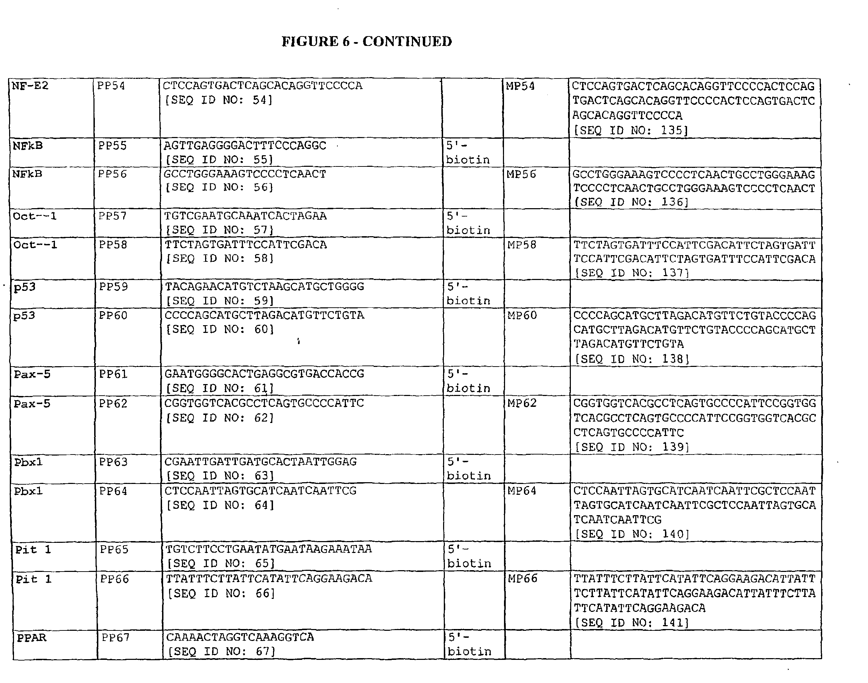

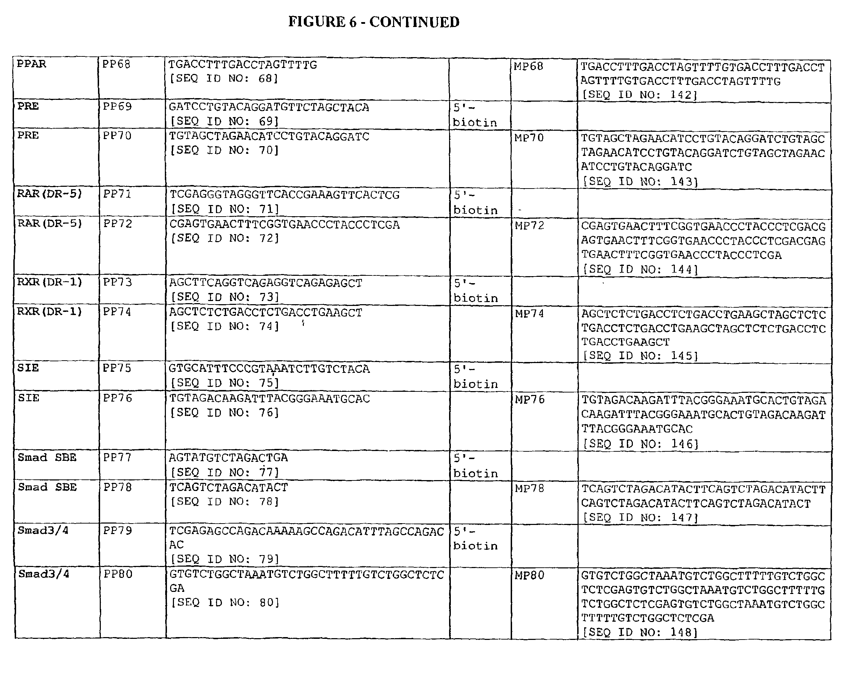

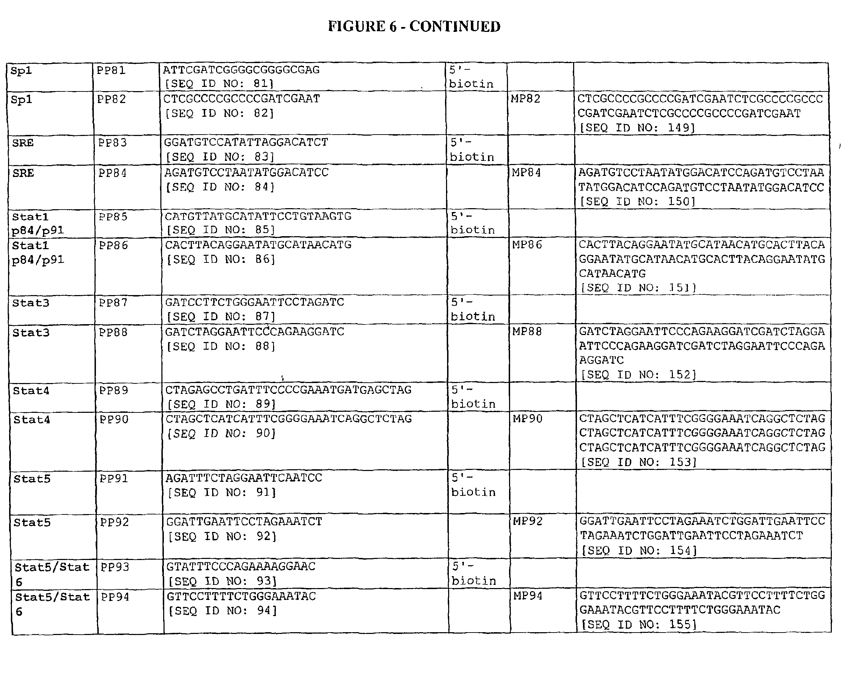

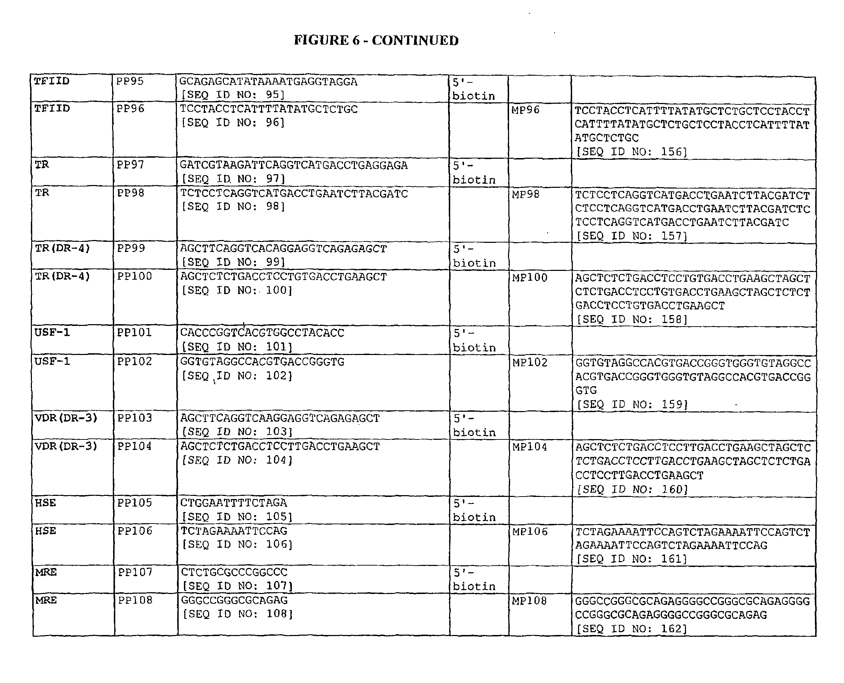

Figure 6 provides the sequences for the probes used to form the transcription factor probe library used in the experiments described in Sections 12-19 herein.

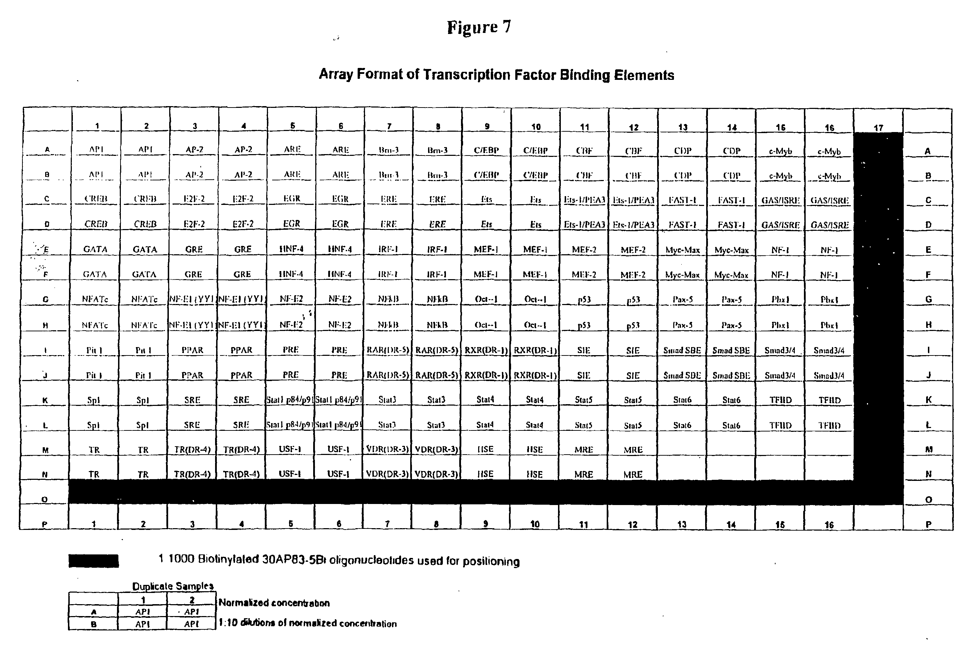

Figure 7 depicts the layout of the array of hybridization probes employed in the experiments described in Sections 12-19 herein.

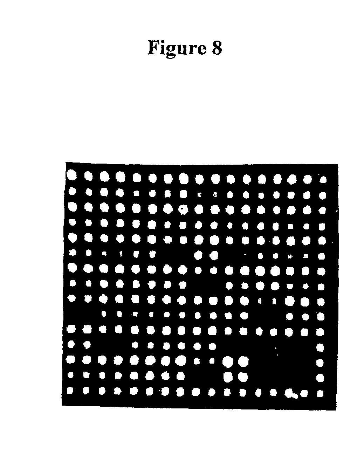

Figure 8 is an image of the array described in regard to Figure 7 when the transcription factor probe library described in Figure 6 is contacted with the array.

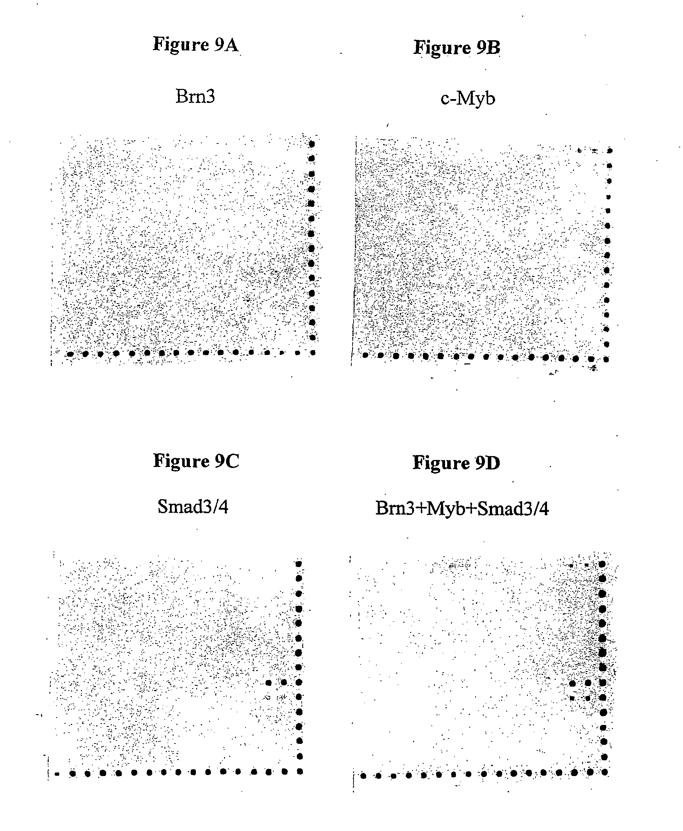

Figure 9A is an image of an array described in regard to Figure 7 that is contacted with transcription factor hybridization probes isolated from transcription factor probe - transcription factor complexes formed when Brn3 transcription factor hybridization probes are contacted with a nuclear extract of HeLa cells.
Figure 9B is an image of an array described in regard to Figure 7 that is contacted with transcription factor hybridization probes isolated from transcription factor probe - transcription factor complexes formed when c-Myb transcription factor hybridization probes are contacted with a nuclear extract of HeLa cells.
Figure 9C is an image of an array described in regard to Figure 7 that is contacted with transcription factor hybridization probes isolated from transcription factor probe - transcription factor complexes formed when Smad3/4 transcription factor hybridization probes are contacted with a nuclear extract of HeLa cells.
Figure 9D is an image of an array described in regard to Figure 7 that is contacted with transcription factor hybridization probes isolated from transcription factor probe - transcription factor complexes formed when Brn3, c-Myb, and Smad3/4 transcription factor hybridization probes are contacted with a nuclear extract of HeLa cells.

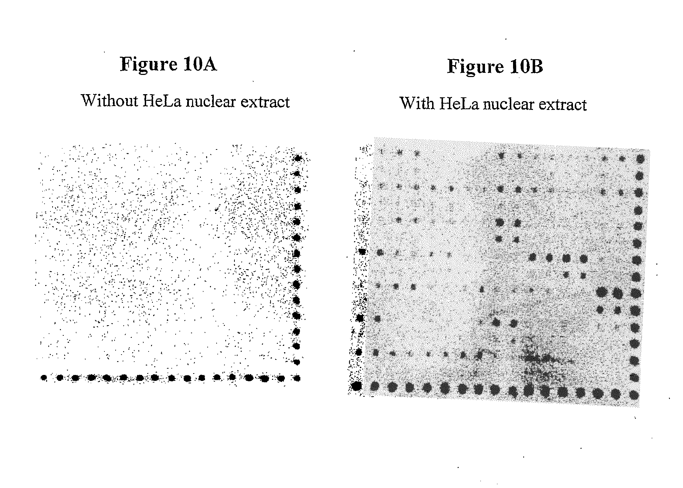

Figure 10A is an image of an array described in regard to Figure 7 that is contacted with transcription factor hybridization probes isolated from transcription factor probe - transcription factor complexes formed when the entire library of transcription factor probes described in regard to Figure 6 are contacted with a control sample that does not contain any transcription factors.
Figure 10B is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of HeLa cells.

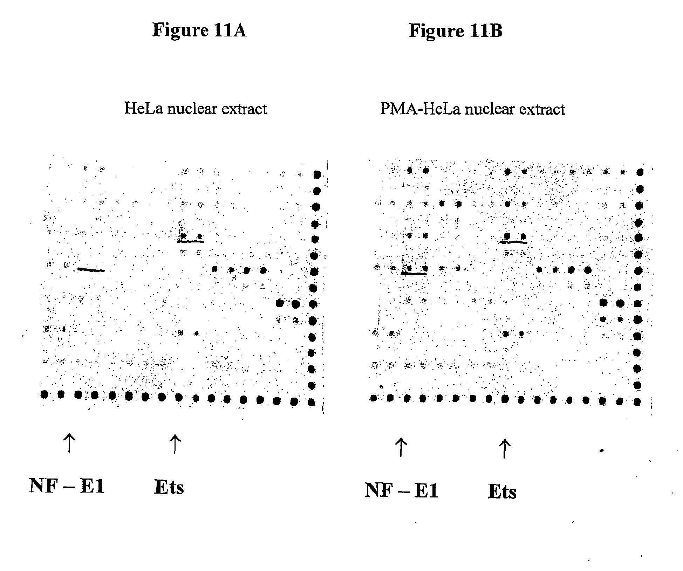

Figure 11A is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of HeLa cells.
Figure 11B is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of PMA-treated HeLa cells.

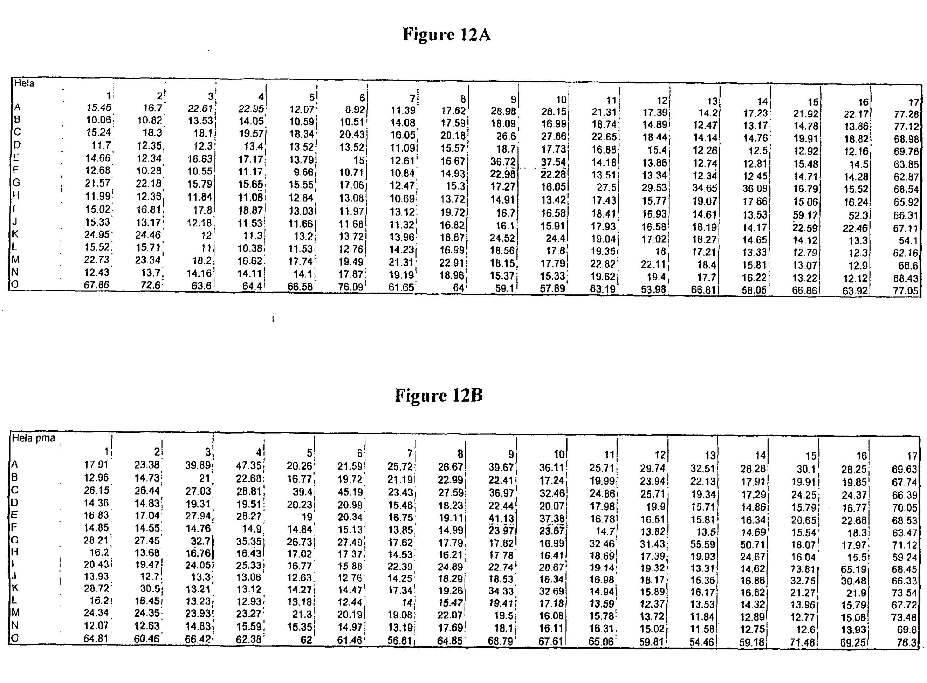

Figure 12A provides a table of the signal intensities of regions of the array shown in Figure 11A.
Figure 12B provides a table of the signal intensities of regions of the array shown in Figure 11B.

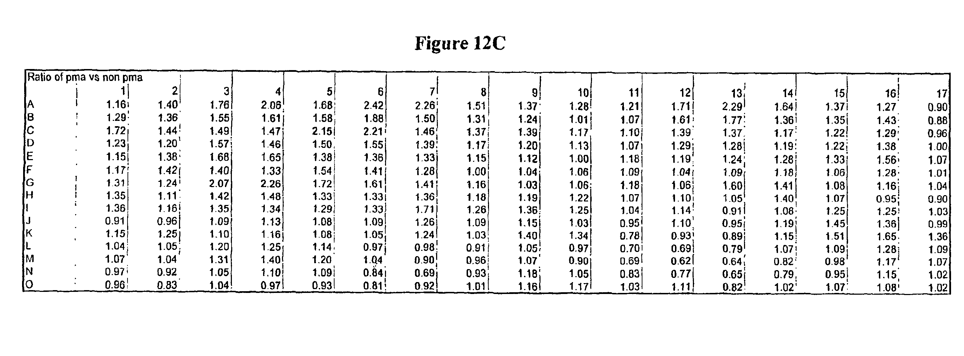

Figure 12C provides a table with the ratios between the intensities of the regions of the arrays shown in Figures 12A and 12B.

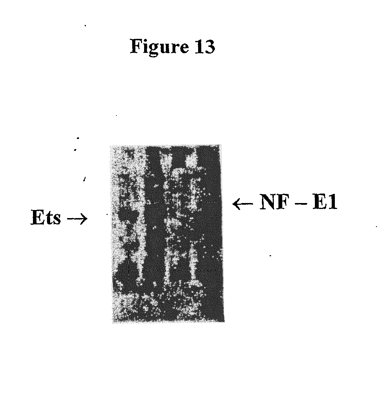

Figure 13 provides an image of a gel shift analysis of Est and NF-E1 performed on HeLa and PMA-treated HeLa cells.

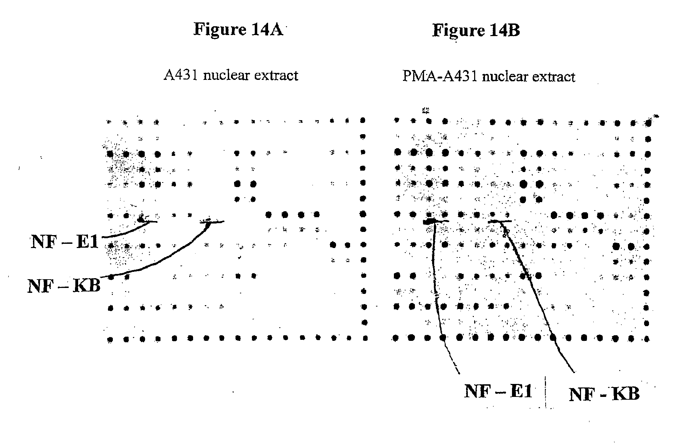

Figure 14A is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of A431 cells.
Figure 14B is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of PMA-treated A431 cells.

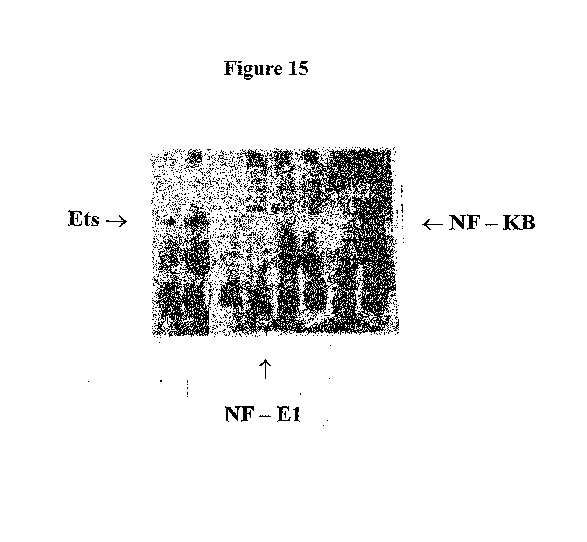

Figure 15 provides an image of a gel shift analysis of Ets, NF-E1, and NF-kB performed on A431 and PMA-treated A431 cells.

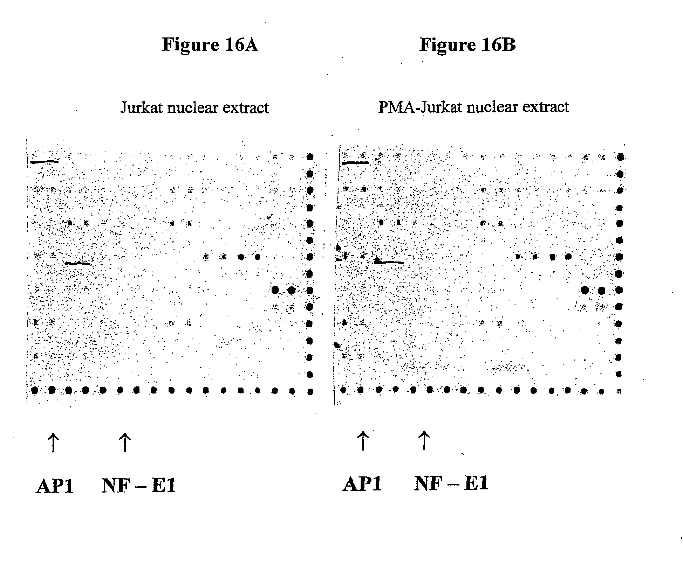

Figure 16A is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of Jurkat cells.
Figure 16B is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of PMA-treated Jurkat cells.

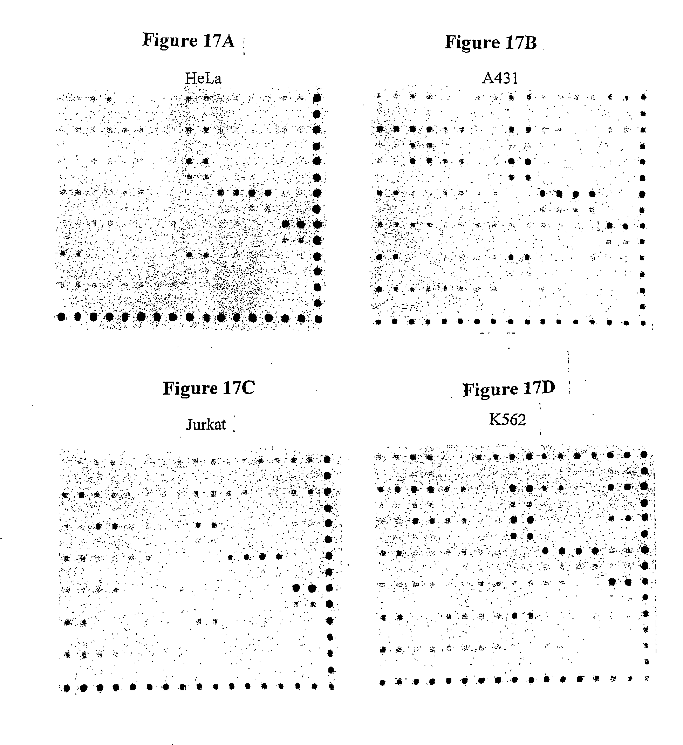

Figure 17A is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of HeLa cells.
Figure 17B is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of A431 cells.
Figure 17C is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of Jurkat cells.
Figure 17D is an image of an array described in regard to Figure 7 that is contacted with transcription factor hybridization probes isolated from transcription factor probe-transcription factor complexes formed when the entire library of transcription factor probes described in regard to Figure 6 are contacted with a nuclear extract of K-562 cells.

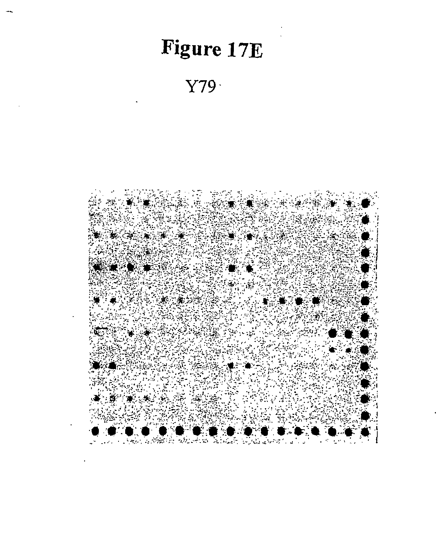

Figure 17E is an image of an array described in regard to Figure 7 that is contacted with transcription factor hybridization probes isolated from transcription factor probe - transcription factor complexes formed when the entire library of transcription factor probes described in regard to Figure 6 are contacted with a nuclear extract of Y79 cells.

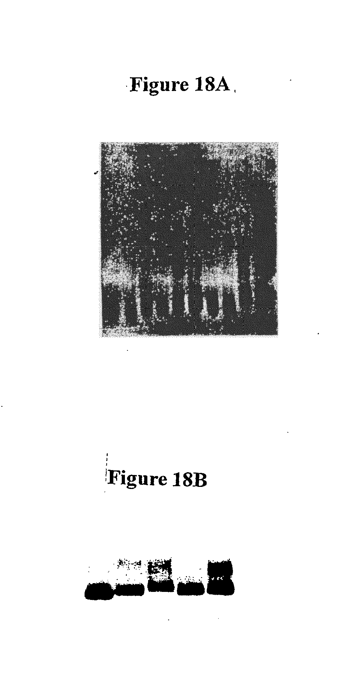

Figure 18A is an image of a polyacrylamide gel from a gel shift analysis for multiple different transcription factors.
Figure 18B is an image of an agarose gel from a gel shift analysis for multiple different transcription factors.

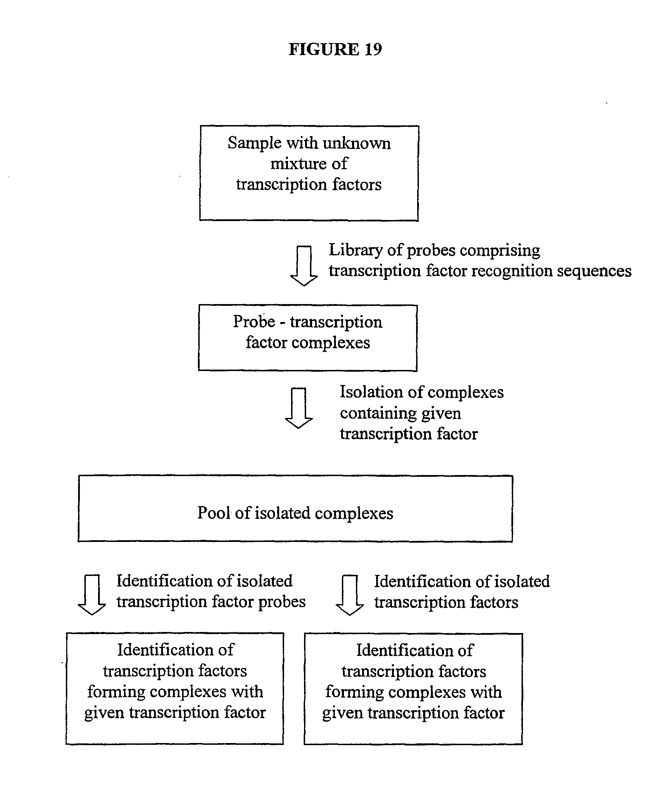

Figure 19 illustrates an embodiment of a method provided according to the present invention for detecting transcription factor - transcription factor complexes.

### DETAILED DESCRIPTION OF THE INVENTION

The present invention relates to rapid and efficient methods for identifying multiple different activated transcription factors in a biological sample at the same time. The methods of the present invention also provide for the quantification of the multiple different activated transcription factors. As will be described herein in greater detail, there are a great many applications that arise from being able to effectively monitor which genes are being expressed at a given time. With the assistance of the methods of the present invention, it is thus possible to rapidly and effectively monitor the levels of expression of multiple different genes at the same time.

The present invention also relates to various kits for use in conjunction with the various methods of the present invention.

Figure 1 provides a general overview of how the present invention detects multiple different activated transcription factors and thus allows the expression of multiple different genes to be simultaneously monitored.

As illustrated, a biological sample is contacted with a library of probes. The biological sample is typically derived from a sample of cells and is preferably a nuclear extract of the cell sample. The sample contains an unknown mixture of activated transcription factors. Which activated transcription factors are present in the sample serves to indicate which genes are currently being expressed.

Information about the DNA binding specificity of transcription factors which one wishes to identify is used to design a library of transcription factor probes. In this regard, it is noted that the present invention relates to the detection, monitoring and optionally quantification of known transcription factors using a library of transcription factor probes whose sequences are known. The probes in the library are preferably double stranded. One of the strands is preferably biotin labeled at the 5' end to facilitate detection.

Each transcription factor probe in the library comprises a DNA sequence that is capable of binding to an activated transcription factor, referred to herein as the probe's recognition sequence. At least the recognition sequence varies within the library of probes such that the probes are capable of binding to a plurality of different activated transcription factors. Due to the high level binding specificity of the transcription factors, each transcription factor probe has binding specificity for a single transcription factor or family of transcription factors.

Because the present invention is used to identify known transcription factors, it is practical to use longer recognition sequences in the probes in the library as compared to what would be practical if a random library were used. As a result, at least 1%, 2%, 3%, 5%, 10%, 20%, 30%, 50% or more of the probes in the library may have recognition sequences greater than 35, 40, 45 or more base pairs in length. By using longer recognition sequences, the probes have greater binding specificity. In addition, the probes have greater binding efficiency to the transcription factors which improves the yield of probe-transcription factor complexes isolated. As a result, the method of the present invention provides a high level of sensitivity for isolating probe - transcription factor complexes, as described further herein, in combination with a high signal to noise ratio.

As a result of contacting the sample with the library of transcription factor probes, complexes are formed between activated transcription factors present in the sample and transcription factor probes in the library which have sequences that match the sequence specificity of the DNA-binding domains of the activated transcription factors. By isolating the transcription factor probe-activated transcription factor complexes, those probes from the library which bind to transcription factors in the sample are isolated.

The isolated transcription factor probes are then identified. Each probe is specific for a different transcription factor. Since only those probes from the library which form a complex with an activated transcription factor will be isolated, identification of which probes are isolated serves to identify which activated transcription factors are present. Since the presence of an activated transcription factor evidences gene expression, the above described method can be used to determine which genes were being expressed at the time the sample was taken based on which activated transcription factors are present.

The design, operation and applications for the present invention will now be described in greater detail.

### 1. Libraries of Transcription Factor Probes

Libraries of transcription factor probes are provided that may be used to detect activated transcription factors according to the present invention. A given library comprises a plurality of double stranded DNA probes where the DNA sequences of the probes vary within the library. The DNA sequences employed in the probes of the libraries preferably have a length between about 10 and 100 base pairs, preferably between about 10 and 75 base *pairs, more* preferably between about 15 and 50. Probes of longer lengths may also be used.

Each probe in the library comprises a recognition sequence which is capable of forming a probe - transcription factor complex with an activated transcription factor. Due to the high level of DNA binding specificity of transcription factors, each transcription factor will typically bind to a different DNA sequence. In some instances, a related family of transcription factors may bind to the same DNA sequence.

By designing the library of probes such that any given probe in the library includes a DNA recognition sequence which a particular activated transcription factor (or a related family of activated transcription factors) will bind to, and does not also include a DNA recognition sequence which other activated transcription factors will bind to, a given probe may be used to identify a single activated transcription factor (or a related family of activated transcription factors).

It is noted that in certain situations, individual probes may bind to more than one activated transcription factor and may nonetheless be used in the library. For example, certain probes may bind to a related family of activated transcription factors. Less than 1:1 binding specificity between probes and activated transcription factors can be readily resolved during analysis of the isolated probes.

The DNA recognition sequences used in the probes in the libraries preferably have a length between about 10 and 100 base pairs, more preferably between about 10 and 75 base pairs, more preferably between about 15 and 50 base pairs, more preferably between about 20 and 40 base pairs, and most preferably a length between about 25 and 35 base pairs.

The optimal length for the recognition sequence and the overall probe may vary somewhat depending on the particular transcription factor. Hence, one may wish to evaluate the optimal length for the recognition sequence and the probe for a given transcription factor using a traditional gel shift assay.

Because the present invention is used to identify known transcription factors, it is practical to use longer recognition sequences in the probes in the library as compared to what would be practical if a random library were used. For example, at least 1%, 2%, 3%, 5%, 10%, 20%, 30%, 50% or more of the probes in the library may have recognition sequences greater than 35, 40, 45 or more base pairs in length. By using longer recognition sequences, the probes have greater binding specificity and greater binding efficiency to the transcription factors. As a result, the method of the present invention provides a high level of sensitivity for isolating probe - transcription factor complexes, as described further herein, in combination with a high signal to noise ratio.

Selection of which DNA recognition sequences to use in a library may be based on the different transcription factors that one wishes to detect in a sample. This, in turn, may depend on the type of organism, cell, or disease state one wishes to identify and/or monitor the gene expression of. It may also depend on the different functionality that one wishes to identify or monitor.

A significant feature of the present invention is the ability to detect multiple different transcription factors at the same time. This ability arises from the number of different DNA recognition sequences used in a library, the number of different DNA recognition sequences relating directly to the number of different transcription factors that the library can be used to detect. A given library of transcription factor probes preferably has at least 2, 3, 5, 10, 20, 50, 100, 250, or more different DNA recognition sequences. The upper limit on the number of different DNA recognition sequences that may be incorporated into a library is limited only by the number of known DNA recognition sequences.

A given library of transcription factor probes may be used to detect gene expression in a single type of cell or organism or may be used to detect gene expression in multiple different types of cells or organisms. When the library is designed to detect gene expression in multiple different types of cells or organisms, the library has DNA recognition sequences for multiple different types of cells or organisms. For example, the library may include DNA recognition sequences for 2, 3, 4, 5 or more different types of cells or organisms. In one embodiment, the library includes DNA recognition sequences for 10, 20, 30, 50, or more different types of cells or organisms.

If the sample comprises cells that may be from one or more different organisms, the DNA recognition sequences used in the library may be for all or some of the different transcription factors expressed by the one or more different organisms. For example, if the library is to be used to classify an unknown type of bacterium, the library may include DNA recognition sequences for multiple different types of bacteria, thereby allowing the library to be used to classify the bacterium.

If the sample comprises cells from a particular organism, the DNA recognition sequences used in the library may be for all or some of the different transcription factors expressed by organism. If the library is to be used to classify an unknown type of cells (i.e., determine whether a growth is malignant), the library may include DNA recognition sequences for multiple different types of cells including the different types of malignant, benign, and normal cell types present in the organism.

If the sample comprises cells of a single cell type, the DNA recognition sequences used in the library may be for all or some of the different transcription factors expressed by that cell type. For example, if one wishes to monitor the expression of only a particular group of genes, such as the genes associated with a particular pathway, the library may include DNA recognition sequences for the transcription factors associated with that group of genes.

As one can see, a myriad of different libraries of probes can be assembled depending on the nature of the sample and the nature of the analysis to be performed. It is noted that different libraries may also be formed when a particularly large number of transcription factors are to be detected or when different binding conditions are needed for different groups of probes.

The probes in the library may optionally further comprise a detectable marker which allows the probe to be detected once isolated from the transcription factor probe - transcription factor complex. Since a wide variety of detection techniques may be used to identify the isolated probes, a similarly wide range of detectable markers may be used in conjunction with those different detection techniques.

The detectable marker may be any marker which can be used to determine the presence or absence of the DNA probes. In a preferred embodiment, the detectable marker is biotin and is preferably attached to one of the 5' ends of probes. Biotin probes have been found to provide a desirable high level of sensitivity.

The detectable marker may also be a dye which can be seen under natural light or with the assistance of an excitation light source to cause fluorescence. In one embodiment, the detectable marker is a fluorescent dye. Examples of fluorescent dyes that may be used include, but are not limited to fluorescein and its derivatives, rhodamine and its derivatives, dansyl, umbeliferone, acridimium, and chemiluminescent molecules such as luciferin and 2,3-dihydrophthalazinediones. The fluorescent dye may also be an energy transfer fluorescent dye.

The detectable marker may also be a molecule which binds to an analytically detectable counterpart. For example, the detectable marker may be covalently attached to or incorporated into the substrate, for example, as taught by Ward, European Patent Application No. 63,879. In such instances, the substrate is detected by adding the analytically detectable counterpart which specifically binds to the substrate, thereby enabling detection of the substrate. Examples of such detectable markers and their analytically detectable counterparts include biotin and either fluorescent or chemiluminescent avidin. Antibodies that bind to an analytically detectable antigen may also be used as the detectable marker. The detectable marker may also be a molecule which, when subjected to chemical or enzymatic modification, becomes detectable such as those disclosed in Leary, et al., Proc. Natl. Acad. Sci. (U.S.A.), 80:4045-4049 (1983).

In certain instances, it may be desirable to employ multiple different detectable markers. For example, if one wishes to classify an unknown type of cell or organism, the library may include DNA probes where different detectable markers are attached to the probes for the different types of cells. Hence, probes for transcription factors expressed by malignant cells may include a first fluorescent dye whereas probes expressed by benign cells may include a second fluorescent dye. In another example, probes for transcription factors expressed by a first type of lung cancer may include a first fluorescent dye whereas probes expressed by of a second, different type of lung cancer may include a second fluorescent dye. This allows one to rapidly visually identify the type of cell based on which detectable markers are present.

When one wishes to compare the gene expression of different groups of cells, multiple libraries may be prepared where each library contains a different detectable marker. For example, a first library labeled with a first detectable marker may be used with a first sample of cells and a second library labeled with a second detectable marker may be used with a second sample of cells. This way, the isolated probes may be analyzed together. As described in Section 6A, when an array format is used for detecting the isolated probes, the use of different detectable markers is particularly advantageous. For example, as shown in Figure 3, transcription factor probes isolated from complexes formed from a first sample (e.g., a control sample) may have a green dye, and transcription factor probes isolated from complexes formed from a second sample (e.g., a test sample) may have a red dye. Regions in the array which are green represent genes which only the cells in the control sample are expressing, regions in the array which are red represent genes which only cells in the test sample are expressing, and regions in the array which are both green and red represent genes which cells in both the control and test samples are expressing.

### 2. Preparation of Sample

Nuclear extracts can be prepared from a sample of cells using the method described by Dignam, J.D., Lebovitz, R.M., and Roeder, R.G. (1983) Accurate transcription initiation by RNA polymerase II in a soluble extract from isolated mammalian nuclei. Nucleic Acids Research 11:1475-1489. Alternatively, a commercially available kit, such as Sigma's Nu-CLEAR Extraction Kit (cat. #N-TRACT) can be used.

### 3. Contracting Cell Sample With Library of Probes

Once a nuclear extract of a sample of cells is prepared, the nuclear extract is contacted with a library of probes and incubated at 15°C for 30 min.

### 4. Isolation of Probe - Transcription Factor Complexes

After contacting the sample with the library of transcription factor probes, any probe - activated transcription factor complexes formed are isolated. Any isolation method which can effectively isolate the complexes may be used. Isolation is preferably performed by a form of size separation, more preferably by electrophoresis, more preferably gel electrophoresis and most preferably gel electrophoresis using an agarose gel.

One of the problems overcome by the present invention is the ability to isolate complexes of DNA probes bound to transcription factors from other probes in the library. Several methods for performing this type of isolation were attempted. However, these methods failed to provide a sufficient yield of probe - transcription factor complexes from the sample.

For example, Applicant attempted to isolate probe - transcription factor complexes by performing an ammonia precipitation. Applicant also attempted to isolate probe - transcription factor complexes by passing the sample through a nitrocellulose filter, the filter serving to immobilize proteins while allowing DNA that is not bound to protein to pass through the filter. Unfortunately, neither of these approaches provided a satisfactory yield of complexes for further characterization of the isolated probes.

Applicant also attempted to isolate probe - transcription factor complexes by using acrylamide gel electrophoresis. Unfortunately, this approach also did not provide a satisfactory yield of complexes. Without being bound by theory, it is believed that this method was hampered by the DNA probes being retained by the acrylamide gel.

Applicant successfully isolated probe - transcription factor complexes from the sample using agaraose gel electrophoresis. Interestingly, despite the fact that agaraose gel electrophoresis does not provide the same quality separation as other forms of gel electrophoresis (e.g., acrylamide gel electrophoresis), the resolution provided using agaraose is more than sufficient to effectively separate the probe - transcription factor complexes. Meanwhile, agarose proved to have satisfactorily low retention of the DNA probes in the complex, thereby allowing the probes to be further characterized.

The separated probe - transcription factor complexes were isolated by removing the band from the gel containing the probe - transcription factor complexes.

It is noted that simultaneous isolation of multiple different probe-transcription factor complexes using a library of DNA probes is a significant departure from traditional gel-shift assays involving the use of a single probe to cause the gel shift of a single transcription factor. [Ausebel, F.M. et al eds (1993) Current Protocols in Molecular Biology Vol.2 Greene Publishing Associates, Inc. and John Wiley and Sons, Inc., New York]. In such prior gel-shift assays, since only a single transcription factor was being detected, no efforts were made to isolate the probe used to cause the gel shift. By contrast, in the present invention, since multiple probes are used in combination it is necessary to isolate the complexes in order to isolate and characterize which of the plurality of probes in the library formed a complex with a transcription factor and are present in the band.

It is also noted that simultaneous isolation of multiple different probe-transcription factor complexes by isolating and characterizing the DNA probes is also a significant departure from U.S. Patent Nos. 6,066,452 and 5,861,246 and PCT Publication No. WO 00/04196 which describe isolation of the nucleic acid binding factors, not the probes.

The following is a detailed description of how one may isolate probe-transcription factor complexes using an agarose gel. It is noted that other separation and isolation methods may be employed without departing from the present invention.

According to one embodiment, a 2% agarose gel in 0.5 X TBE is prepared. Preferably, 8 mm-wide combs are used. Each sample is mixed with 2 µl of a gel loading buffer. Table 1 provides an embodiment of a gel loading buffer that may be used.

**TABLE 1**

| **Gel shift loading buffer:** |
|---|
| 0.25 X TBE buffer: 60% |
| Glycerol: 40% |
| Bromophenol blue: 0.2% (w/v) |

Add 2 ul of gel shift loading buffer and load into a 0.8 cm width lane of 2% agarose gel in 0.5% TBE. The resulting agarose gel is then run in 0.5% TBE at 120V for 16 min. The gel area containing that contains the protein/DNA complex, which will be above the blue dye and below the gel lane, is excised and transferred to a 1.5 mL tube.

### 5. Separation of Transcription Factor Probes From Transcription Factor - Probe Complexes

Once the transcription factor probe - transcription factor complexes are separated from other proteins and DNA in the sample, for example, as described in Section 4, the transcription factor probes are separated from the excised portion of the gel.

The following is a detailed description of how one may isolate probe-transcription factor complexes from an excised portion of an agarose gel. It is noted that other isolation techniques may be employed without departing from the present invention.

It is noted that the following steps describing the isolation of the DNA probes is specifically designed for CLONTECH's NucleoTrap Kit. If another commercially available gel extraction kit is employed, the steps should be modified in accordance with that manufacturer's instructions.

1.0 mL of NT1 solution from CLONTECH's NucleoTrap Kit is added to the excised portion of the gel. The mixture is then incubated at 50°C until the gel is totally dissolved. The tube is preferably periodically gently inverted in order to mix the contents until the gel is dissolved.

6 µl of beads from a commercially available gel extraction kit is then preferably added and the resulting mixture is incubated at room temperature for 10 min. The tube is preferably periodically gently inverted every 2-3 minutes.

The tube is then microfuged at 10,000 rpm for 30 sec. The resulting supernatant is carefully removed and the pellet resuspended in 150 µl of NT2 solution from CLONTECH's NucleoTrap Kit.

The pellet is then microfuged at 10,000 rpm for 30 sec. The resulting supernatant is then carefully removed and the pellet resuspended in 150 µl of NT3 solution from CLONTECH's NucleoTrap Kit. The pellet is then microfuged at 10,000 rpm for 30 sec. The supernatant is again removed and the pellet is allowed to air-dry for 10 min.

50 µl of dH₂O is then added to resuspend the pellet. The resulting mixture is incubated at room temperature for 5 min. The mixture is then gently shaken and incubated for another 5 min.

The resulting mixture is then microfuged at 10,000 rpm for 1 min. The supernatant is transferred to a fresh 1.5 ml tube. The isolated supernatant contains the DNA probes that are bound to proteins. The isolated supernatant is preferably stored on ice until proceeding to a characterization of the DNA probes.

### 6. Identifying Isolated Transcription Factor Probes

A variety of different methods may be used to identify which of the transcription factor probes from the library are present in the isolated probe-transcription factor complexes. These methods preferably also allow for the amount of transcription factor probes isolated to also be quantified. By identifying which transcription factor probes form complexes, one is able to determine which transcription factors are present in an activated form in the sample, the presence of an activated transcription factor evidencing expression of the gene associated with the transcription factor. By quantifying the amount of each transcription factor probe that forms a complex, one is able to determine the amount of each transcription factor present and hence the level of expression of the gene associated with that transcription factor.

One method that may be used to identify which of the transcription factor probes from the library are present in the isolated probe - transcription factor complexes is mass spectroscopy. According to this method, the length and composition of each probe can be determined. Therefore, the analyzed results show whether a specific probe is existing in the complexes and the interactions between transcription factors and binding probes can be determined.

Another method that may be used to identify which of the transcription factor probes from the library are present in the isolated probe - transcription factor complexes is based on size separation. According to this method, one varies the length of the probes in the transcription factor probe library so that it is possible to resolve the different sized probes based on a size-based separation. For example, electrophoresis may be performed in order to separate the probes based on size. Such size-based DNA separations are traditionally done with high level specificity for DNA sequencing. By identifying which transcription factor probes are present based on the size-based separation, one can determine which activated transcription factors are present and can also quantify the amount of each activated transcription factor.

Yet another method for identifying which of the probes from the transcription factor probe library formed complexes involves hybridization of the transcription factor probes with a hybridization probe comprising a complement to the transcription factor probes recognition sequence. According to this method, detection of a particular transcription factor probe is accomplished by detecting the formation of a duplex between the transcription factor probe and a hybridization probe comprising a complement to the transcription factor probe's recognition sequence.

A wide variety of assays have been developed for performing hybridization assays and detecting the formation of duplexes that may be used in the present invention. For example, hybridization probes with a fluorescent dye and a quencher where the fluorescent dye is quenched when the probe is not hybridized to a target and is not quenched when hybridized to a target oligonucleotide may be used. Such fluorescer-quencher probes are described in, for example, U.S. Patent No. 6,070,787 and S. Tyagi et al., "Molecular Beacons: Probes that Fluoresce upon Hybridization", Dept. of Molecular Genetics, Public Health Research Institute, New York, N. Y., Aug. 25, 1995. By attaching different fluorescent dyes to different hybridization probes, it is possible to determine which transcription factor probes from the library formed complexes based on which fluorescent dyes are present (e.g, fluorescent dye and quencher on hybridization probe or fluorescent dye on hybridization probe and quencher on transcription factor probe). Applicant notes that one may also attach different fluorescent dyes to different transcription factor probes and use a change in fluorescence due to hybridization to a hybridization probe to determine which transcription factor probes are present (e.g, fluorescent dye and quencher on transcription factor probe or fluorescent dye on transcription factor probe and quencher on hybridization probe).

A difficulty, however, arises when using multiple different fluorescer to detect multiple different transcription factor probes. Namely, there is a limited number of different fluorescers that may be spectrally resolved. As a result, a limited number of different transcription factors can be detected at the same time, for example only as many as five to ten.

### A. Hybridization Arrays For Detecting Isolated Transcription Factor Probes

A preferred assay for detecting the formation of duplexes between transcription factor probes and hybridization probes comprising their complements involves the use of an array of hybridization probes immobilized on a solid support. The hybridization probes comprise sequences that are complementary to at least a portion of the recognition sequences of the transcription factor probes and thus are able to hybridize to the different probes in a transcription factor probe library.

In order to improve enhance the sensitivity of the hybridization array, the immobilized probes preferably provide at least 2, 3, 4 or more copies of at least a portion of the recognition sequence incorporated into the transcription factor probes.

According to the present invention, the hybridization probes immobilized on the array preferably are at least 25 nucleotides in length, more preferably at least 30, 40 or 50 nucleotides in length. The immobilized hybridization probes may be 50, 75, 100 nucleotides or longer in length. In one preferred embodiment the immobilized probes are between 50 and 100 nucleotides in length.

By immobilizing hybridization probes on a solid support which comprise one or more copies of a complement to at least a portion of the recognition sequences of the transcription factor probes, the hybridization probes serve as immobilizing agents for the transcription factor probes, each different hybridization probe being designed to selectively immobilize a different transcription factor probe.

Figure 2 illustrates an array of hybridization probes attached to a solid support where different hybridization probes are attached to discrete, different regions of the array. Each different region of the array comprises one or more copies of a same hybridization probe which incorporates a sequence that is complementary to a recognition sequence of a transcription factor probe. As a result, the hybridization probes in a given region of the array can selectively hybridize to and immobilize a different transcription factor probe based on the transcription factor probe's recognition sequence.

By detecting which regions the isolated transcription factor probes hybridize to on the array, one can determine which activated transcription factors are present in the sample and can also quantify the amount of each activated transcription factor.

These arrays can be designed and used to study transcription factor activation in a variety of biological processes, including cell proliferation, differentiation, transformation, apoptosis, drug treatment, and others described herein.

Numerous methods have been developed for attaching hybridization probes to solid supports in order to perform immobilized hybridization assays and detect target oligonucleotides in a sample. Numerous methods and devices are also known in the art for detecting the hybridization of a target oligonucleotide to a hybridization probe immobilized in a region of the array. Examples of such methods and device for forming arrays and detecting hybridization include, but are not limited to those described in U.S. Patent Nos. 6,197,506, 6,045,996, 6,040,138, 5,424,186, 5,384,261.

Several modifications may be made to the hybridization arrays known in the art in order to customize the hybridization arrays for use in detecting activated transcription factors through the characterization of isolated transcription factor probes which form a complex with the activated transcription factors.

Since the hybridization probe arrays for use in the methods of the present invention are designed to hybridize to the probes in the transcription factor probe library by comprising a sequence that is complementary to the transcription factor recognition sequence, the composition of the hybridization probes in the array should complement the recognition sequences of the probes in the transcription factor probe library. As discussed in Section 1, a variety of different libraries of transcription factor probes are provided that may be used to detect activated transcription factors according to the present invention.

Selection of the sequences used in the hybridization probes may be based on the different transcription factors that one wishes to detect in a sample. This, in turn, may depend on the type of organism, cell, or disease state one wishes to identify and/or monitor the gene expression of.

A significant feature of the present invention is the ability to detect multiple different transcription factors at the same time. This ability arises from the number of different DNA recognition sequences used in a library, the number of different DNA recognition sequences relating directly to the number of different transcription factors that the library can be used to detect. A given array of hybridization probes preferably has complements for at least 2, 3, 5, 10, 20, 30, 50, 100, 250 or more different DNA recognition sequences. The upper limit on the number of different DNA recognition sequences that the array of hybridization probes may detect is limited only by the number of known DNA recognition sequences and hence the number of known complements to the DNA recognition sequences.

A given array of hybridization probes may be used to detect gene expression in a single type of cell or organism or may be used to detect gene expression in multiple different types of cells or organisms. When the array is designed for use with a library designed to detect gene expression in multiple different types of cells or organisms, the array may include complements to DNA recognition sequences for multiple different types of cells or organisms. For example, the array may include complements to DNA recognition sequences for 2, 3, 4, 5 or more different types of cells or organisms. In one embodiment, the array may include complements to DNA recognition sequences for 10, 20, 30, 50, or more different types of cells or organisms.

If the sample to be analyzed comprises cells that may be from one or more different organisms, the DNA recognition sequences used in the library may be for all or some of the different transcription factors expressed by the one or more different organisms. For example, if library is to be used to classify an unknown type of bacterium, the library may include DNA recognition sequences for multiple different types of bacteria, thereby allowing the library to be used to classify the bacterium. Accordingly, the array used in combination with the library would include complements to DNA recognition sequences for multiple different types of bacteria.

If the sample comprises cells from a particular organism, the DNA recognition sequences used in the library may be for all or some of the different transcription factors expressed by organism. Accordingly, the array used in combination with the library would include complements to DNA recognition sequences for the different transcription factors expressed by organism.

If the library is to be used to classify an unknown type of cells (i.e., determine whether a growth is malignant), the library may include DNA recognition sequences for multiple different types of cells including the different types of malignant, benign, and normal cell types present in the organism. Accordingly, the array used in combination with the library would include complements to DNA recognition sequences for the multiple different types of cells.

If the sample comprises cells of a single cell type, the DNA recognition sequences used in the library may be for all or some of the different transcription factors expressed by that cell type. Accordingly, the array used in combination with the library would include complements to the recognition sequences for all or some of the different transcription factors expressed by that cell type.

### i. Procedure for Performing Hybridization Using Array

Provided below is a description of a procedure that may be used to hybridize isolated transcription factor probes to a hybridization array. It is noted that the below procedure may be varied and modified without departing from other aspects of the invention.

An array membrane having hybridization probes attached for the transcription factor probes is first placed into a hybridization bottle. The membrane is then wet by filling the bottle with deionized H₂O. After wetting the membrane, the water is decanted. Membranes that may be used as array membranes include any membrane to which a hybridization probe may be attached. Specific examples of membranes that may be used as array membranes include, but are not limited to NYTRAN membrane (Schleicher & Schuell), BIODYNE membrane (Pall), and NYLON membrane (Roche Molecular Biochemicals).

5 ml of prewarmed hybridization buffer is then added to each hybridization bottle containng an array membrane. The bottle is then placed in a hybridization oven at 42°C for 2 hr. An example of a hybridization buffer that may be used is EXPHYP by Clonetech.

After incubating the hybridization bottle, a thermal cycler may be used to denature the hybridization probes by heating the probes at 90°C for 3 min, followed by immediately chilling the hybridization probes on ice.

The isolated probe - transcription factor complexes are then added to the hybridization bottle. Hybridization is preferably performed at 42°C overnight.

After hybridization, the hybridization mixture is decanted from the hybridization bottle. The membrane is then washed repeatedly.

In one embodiment, washing includes using 60 ml of a prewarmed first hybridization wash which preferably comprises 2X SSC/0.5% SDS. The membrane is incubated in the presence of the first hybridization wash at 42°C for 20 min with shaking. The first hybridization wash solution is then decanted and the membrane washed a second time. A second hybridization wash, preferably comprising 0.1X SSC/0.5% SDS is then used to wash the membrane further. The membrane is incubated in the presence of the second hybridization wash at 42°C for 20 min with shaking. The second hybridization wash solution is then decanted and the membrane washed a second time.

### ii. Procedure for Detecting Array Hybridization

The following describes a procedure that may be used to detect isolated transcription factor probes isolated on the hybridization array. It is noted that each membrane should be separately hybridized, washed and detected in separate containers in order to prevent cross contamination between samples. It is also noted that it is preferred that the membrane is not allowed to dry during detection.

According to the procedure, the membrane is carefully removed from the hybridization bottle and transferred to a new container containing 30 ml of 1X blocking buffer. The dimensions of each container is preferably about 4.5" x 3.5", equivalent in size to a 200 µL pipette-tip container. Table 2 provides an embodiment of a blocking buffer that may be used.

**TABLE 2**

| **1X Blocking Buffer:** |
|---|
| Blocking reagent: 1% |
| 0.1 M Maleic acid |
| 0.15M NaCl |
| Adjusted with NaOH to pH 7.5 |

It is noted that the array membrane may tend to curl adjacent its edges. It is desirable to keep the array membrane flush with the bottom of the container.

The array membrane is incubated at room temperature for 30 min with gentle shaking. 1 ml of blocking buffer is then transferred from each membrane container to a fresh 1.5 ml tube. 3 µl of Streptavidin-AP conjugate is then added to the 1.5 ml tube and is mixed well. The contents of the 1.5 ml tube is then returned to the container and the container is incubated at room temperature for 30 min.

The membrane is then washed three times at room temperature with 40 ml of 1X detection wash buffer, each 10 min. Table 3 provides an embodiment of a 1X detection wash buffer that may be used.

**TABLE 3**

| **1 X Detection wash buffer:** |
|---|
| 10 mM Tris-HCl, pH 8.0 |
| 150 mM NaCl |
| 0.05% Tween-20 |

30 ml of 1X detection equilibrate buffer is then added to each membrane and the combination is incubated at room temperature for 5 min. Table 4 provides an embodiment of a 1X detection equilibrate buffer that may be used.

**TABLE 4**

| **1 X Detection equilibrate buffer:** |
|---|
| 0.1 M Tris-HCl pH 9.5 |
| 0.1 M NaCl |

The resulting membrane is then transferred onto a transparency film. 3 ml of CPD-*Star* substrate, produced by Applera, Applied Biosystems Division, is then pipetted onto the membrane.

A second transparency film is then placed over the first transparency. It is important to ensure that substrate is evenly distributed over the membrane with no air bubbles. The sandwich of transparency films are then incubated at room temperature for 5 min.

The *CPD-Star* substrate is then shaken off and the films are wiped. The membrane is then exposed to Hyperfilm ECL, available from Amersham-Pharmercia. Alternatively, a chemiluminescence imaging system may be used such as the ones produced by ALPHA INNOTECH. It may be desirable to try different exposures of varying lengths of time (e.g., 2-10 min).

The hybridization array may be used to obtain a quantitative analysis of the amount of transcription factor probe present. For example, if a chemiluminescence imaging system is being used, the instructions that come with that system's software should be followed. If Hyperfilm ECL is used, it may be necessary to scan the film to obtain numerical data for comparison.

### iii. Normalization of Data From Array Hybridization

One of the advantages provided by array hybridization for detecting isolated transcription factor probes is the ability to simultaneously analyze whether multiple different activated transcription factors are present.

A further advantage provided is that the system allows one to compare a quantification of multiple different activated transcription factors between two or more samples. When two or more arrays from multiple samples are compared, it is desirable to normalize them.

In order to facilitate normalization of the arrays, an internal standard may be used so that the intensity of detectable marker signals between arrays can be normalized. In certain instances, the internal standard may also be used to control the time used to develop the detectable marker.

In one embodiment, the internal standard for normalization is biotinylated DNA which is spotted on a portion of the array, preferably adjacent one or more sides of the array. For example, biotin-labeled ubiquitin DNA may be positioned on the bottom line and last column of the array. In order to normalize two or more arrays for comparison of results, the exposure time for each array should be adjusted so that the signal intensity in the region of the biotinylated DNA is approximately equivalent on both arrays.

### 7. Use of Multiple Libraries In Combination To Compare Gene Expression Between Different Samples

When an array format is used for detecting the isolated probes, it may be desirable to use multiple libraries labeled with different detectable markers in order to facilitate comparison between samples. For example, as shown in Figure 3, probes from a first sample (e.g., a control sample) may have a green dye, and probes from a second sample (e.g., a test sample) may have a red dye. Both probes are separated from their bound complexes, mixed, and hybridized to a single array. Green spots in the array represent genes which only the cells in the control sample are expressing, and red spots in the array represent genes which only cells in the test sample are expressing. When both dyes hybridize to the same spot in an equal amount, the balanced mixture of green and red appears as yellow in the array, representing genes which cells in both the control and test samples are expressing.

One embodiment of this application of the present invention thus relates to a method for comparing gene expression between a test sample and a control sample, the method comprising forming transcription factor probe - activated transcription factor complexes using the test sample and a first library of transcription factor probes having a first detectable marker; forming transcription factor probe - activated transcription factor complexes using the control sample and a second library of transcription factor probes having the same nucleic acid sequences as the first library but having a second, different detectable marker; isolating the transcription factor probes from the first library which formed complexes involving transcription factors from the test sample; isolating the transcription factor probes from the second library which formed complexes involving transcription factors from the control sample; and detecting the isolated transcription factor probes from the first and second libraries using a same hybridization array.

A kit comprising the first and second library of transcription factor probes is described herein. The kit may optionally further include a hybridization array comprising complements to the transcription factor probes. The kit may also include instructions for isolating the transcription factor probe - activated transcription factor complexes using agarose gel.

### 8. Applications For Monitoring Gene Expression Via Detection Of Activated Transcription Factors

By better understanding which cells express which genes and how different conditions influence gene expression, fundamental questions of biology can be answered. Thus, by being able to rapidly and efficiently detect multiple activated transcription factors at the same time, the present invention avails itself to numerous valuable applications relating to the monitoring of gene expression. Some of these applications are described herein. Other applications will be apparent to those of ordinary skill.

### a. Characterization of Cell Type

By detecting and optionally quantifying which activated transcription factors are present in a cell sample, the methods of the present invention allow one to identify which genes are being expressed and to what extent each gene is being expressed. Different types of cells for a particular organism will express different genes. As a result, the present invention allows one to rapidly characterize a cell type based on which activated transcription factors are present and at what levels.

One embodiment of this application of the present invention thus relates to a method for characterizing a cell type, the method comprising forming transcription factor probe - activated transcription factor complexes using a test sample and a library of transcription factor probes comprising recognition sequences characteristic of different types of cells; isolating the transcription factor probes from the library which formed complexes involving transcription factors from the test sample; and detecting the isolated transcription factor probes using a hybridization array comprising sequences complementary to the transcription factor probes in the library.

A further embodiment of this application of the present invention relates to a library of transcription factor probes and hybridization array comprising compliments to the library of transcription factor probes are provided where the transcription factor probes comprise recognition sequences from multiple different cell types. A kit is also provided that comprises both the library of probes and the hybridization array. The kit may also include instructions for isolating the transcription factor probe - activated transcription factor complexes using an agarose gel, either in combination with the library, the hybridization array, or both.

It is noted that different organisms will also express different activated transcription factors. Characterizing the mixture of different activated transcription factors expressed by a particular organism (e.g., a culture of bacteria) can be used to identify the particular organism. This application of the method of the present invention may be particularly useful for rapidly characterizing microbes such as bacteria and diseased tissue.

One embodiment of this application of the present invention thus relates to a method for characterizing an organism, the method comprising forming transcription factor probe - activated transcription factor complexes using a test sample and a library of transcription factor probes comprising recognition sequences characteristic of different organisms; isolating the transcription factor probes from the library which formed complexes involving transcription factors from the test sample; and detecting the isolated transcription factor probes using a hybridization array comprising sequences complementary to the transcription factor probes in the library.

A further embodiment of this application of the present invention relates to a library of transcription factor probes and hybridization array comprising compliments to the library of transcription *factor* probes are provided where the transcription factor probes comprise recognition sequences from multiple different organisms. A kit is also provided that comprises both the library of probes and the hybridization array. The kit may also include instructions for isolating the transcription factor probe - activated transcription factor complexes using an agarose gel, either in combination with the library, the hybridization array, or both.

It is noted that the mixture of different activated transcription factors expressed by different cell types or organisms may be used according to the present invention as a form of an expression signature for that cell type. Figure 2 illustrates an array detection format. The pattern formed by the detectable markers [cubes] in Figure 2 can be used as a visual fingerprint of the expression signature and can be used to identify a particular cell type or organism based on that visual fingerprint. In this regard, it is envisioned that an array may be developed with a great multiplicity of immobilizing agents for different transcription factor probes. It is noted that the number of cubes shown in the figure is employed to reflect signal intensity.

The array may include immobilizing agents for transcription factor probes for different cell types and/or for different organisms. By comparing the array pattern to a standard for a particular cell type or organism, the cell type or organism can be rapidly determined.

### b. Determining the Functions of Different Genes

Despite the fact that each cell in the human body contains the same set of genes, the human body is comprised of a wide diversity of different cell types that work in concert to form the human body. The wide diversity of cell types present in the human body and other multicellular organisms is due to variations between cells regarding which genes are expressed, the level at which the genes are expressed, and the conditions under which the genes are expressed. The present invention provides the unique ability of rapidly determining which of a great number of genes are expressed by numerous different cell types. By being able to determine which genes are expressed by which cell types, the functions of different genes can be deduced.

### c. Diagnosis of Disease States

Certain disease states may be caused and/or characterizable by certain genes being expressed or not expressed as compared to normal cells. Other disease states may result from and/or be characterizable by certain genes being transcribed at different levels as compared to normal cells.

By being able to rapidly monitor the expression levels of multiple different genes, the present invention provides an accurate method for diagnosing certain disease states known to be associated with the expression non-expression, reduced expression, and/or elevated expression of one or more genes. Conversely, by comparing the expression non-expression, reduced expression, and/or elevated expression of one or more genes in normal and abnormal cells, present invention facilitates the association of one or more genes with certain disease states. By understanding that a particular disease state is caused by a different expression (higher or lower) of one or more proteins, it should be possible to remedy the disease state by increasing or decreasing the expression of the one or more proteins, by administering the one or more proteins or, if particular proteins are overexpressed, by inhibiting the one or more proteins.

One embodiment of this application of the present invention thus relates to a method for diagnosing a disease state of a sample of cells, the method comprising forming transcription factor probe - activated transcription factor complexes using the sample of cells and a library of transcription factor probes comprising different transcription factor recognition sequences; isolating the transcription factor probes from the library which form complexes involving transcription factors from the sample; detecting the isolated transcription factor probes using a hybridization array comprising sequences complementary to the transcription factor probes in the library; and diagnosing a presence of a disease state based on which transcription factors are activated in the cell sample as identified by which transcription factor probes are isolated.

### d. Compound Screening For Drug Candidates

Being able to monitor transcription factor activity for multiple different transcription factors at the same time is of great importance to developing a better understanding of different roles that various transcription factors play. In addition, monitoring multiple different transcription factors at the same time allows one to rapidly screen for compounds that influence transcription factor activity, referred to herein as a "transcription factor modulator."

The present invention may thus be used as a high throughput screening assay for transcription factor modulators that either up- or down-regulate genes by influencing the synthesis and activation of transcription factors for those genes.

By having a further understanding of what compounds modulate transcription factor activity, such compounds may be more effectively used for *in vitro* modification of signal transduction, transcription, splicing, and the like, e.g., as tools for recombinant methods, cell culture modulators, etc. More importantly, such compounds can be used as lead compounds for drug development for a variety of conditions, including as antibacterial, antifungal, antiviral, antineoplastic, inflammation modulatory, or immune system modulatory agents. Accordingly, being able to monitor transcription factor activity for multiple different factors has great use for screening compounds to identify lead compounds for pharmaceutical or other applications.

Indeed, because gene expression is fundamental in all biological processes, including cell division, growth, replication, differentiation, repair, infection of cells, etc., the ability to monitor transcription factor activity and identify compounds which modulator their activity can be used to identify drug leads for a variety of conditions, including neoplasia, inflammation, allergic hypersensitivity, metabolic disease, genetic disease, viral infection, bacterial infection, fungal infection, or the like. In addition, compounds which specifically target transcription factors in undesired organisms such as viruses, fungi, agricultural pests, or the like, can serve as fungicides, bactericides, herbicides, insecticides, and the like. Thus, the range of conditions that are related to transcription factor activity includes conditions in humans and other animals, and in plants, e.g., for agricultural applications.

As used herein, the term "transcription factor modulator" refers to any molecule or complex of more than one molecule that affects the regulatory region. The present invention contemplates screens for synthetic small molecule agents, chemical compounds, chemical complexes, and salts thereof as well as screens for natural products, such as plant extracts or materials obtained from fermentation broths. Other molecules that can be identified using, the screens of the invention include proteins and peptide fragments, peptides, nucleic acids and oligonucleotides (particularly triple-helix-forming oligonucleotides), carbohydrates, phospholipids and other lipid derivatives, steroids and steroid derivatives, prostaglandins and related arachadonic acid derivatives, etc.

Existing methods for monitoring gene expression typically monitor down-stream expression processes by measuring mRNA or the resulting gene product. However, why a particular mRNA or protein is expressed at higher or lower levels is not revealed by these methods. This is because a given compound can influence the formation of a transcription factor, influence the activation of the transcription factor, interact with the activated transcription factor, interact with the regulatory element to which the transcription factor binds, or interact with the mRNA that is produced.

By contrast, because the present invention is specific to detecting activated transcription factors, the present invention can be effectively used to screen for drugs that have a mechanism of action directly related to the expression and/or activation of transcription factors.

It should be noted that methods exist for measuring a transcription factor in a sample. However, because such methods detect the protein itself, they are unable to determine whether the transcription factor is activated, i.e., it is capable of binding to a regulatory element. By being able to detect whether multiple different transcription factors are activated, the present invention, when used in combination with an assay for detecting the amount of activated and unactivated transcription factor, allows one to evaluate specifically how a given compound influences the activation of different transcription factors.

The present invention may be used to screen large chemical libraries for modulator activity for multiple different transcription factors. For example, by exposing cells to different members of the chemical libraries, and performing the methods of the present invention, one is able to screen the different members of the library relative to multiple different transcription factors at the same time.

It will be appreciated that there are many suppliers of chemical compounds, including Sigma (St. Louis, Mo.), Aldrich (St. Louis, Mo.), Sigma-Aldrich (St. Louis, Mo.), Fluka Chemika-Biochemica Analytika (Buchs Switzerland) and the like.

In one preferred embodiment, high throughput screening involves testing a combinatorial library containing a large number of potential modulator compounds. A combinatorial chemical library may be a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length (i.e., the number of amino acids in a polypeptide compound). Millions of chemical compounds can be synthesized through such combinatorial mixing of chemical building blocks.

Such combinatorial libraries are then screened to identify those library members (particular chemical species or subclasses) that modulate one or more transcription factors. The compounds thus identified can serve as conventional "lead compounds" or can themselves be used as potential or actual therapeutics for the one or more transcription factors whose activities the compounds modulate.

Preparation and screening of combinatorial libraries is well known to those of skill in the art. Such combinatorial libraries include, but are not limited to, peptide libraries (e.g., U.S. Pat. No. 5,010,175, Furka, Int. J. Pept. Prot. Res. 37:487-493 (1991) and Houghton et al., Nature 354:84-88 (1991)). Other chemistries for generating chemical diversity libraries can also be used. Such chemistries include, but are not limited to: peptoids (PCT Publication No. WO 91/19735), encoded peptides (PCT Publication WO 93/20242), random bio-oligomers (PCT Publication No. WO 92/00091), benzodiazepines (U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with .beta.-D-glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries (see, Ausubel, Berger and Sambrook, all supra), peptide nucleic acid libraries (see, e.g., U.S. Pat. No. 5,539,083), antibody libraries (see, e.g., Vaughn et al., Nature Biotechnology, 14(3):309-314 (1996) and PCT/US96/10287), carbohydrate libraries (see, e.g., Liang et al., Science, 274:1520-1522 (1996) and U.S. Pat. No. 5,593,853), small organic molecule libraries (see, e.g., benzodiazepines, Baum C&EN, Jan 18, page 33 (1993); isoprenoids, U.S. Pat. No. 5,569,588; thiazolidinones and metathiazanones, U.S. Pat. No. 5,549,974; pyrrolidines, U.S. Pat. Nos. 5,525,735 and 5,519,134; morpholino compounds, U.S. Pat. No. 5,506,337; benzodiazepines, 5,288,514, and the like).

Devices for the preparation of combinatorial libraries are also commercially available (see, e.g., 357 MPS, 390 MPS, Advanced Chem Tech, Louisville Ky., Symphony, Rainin, Woburn, Mass., 433A Applied Biosystems, Foster City, Calif., 9050 Plus, Millipore, Bedford, Mass.). In addition, numerous combinatorial libraries are themselves commercially available (see, e.g., ComGenex, Princeton, N.J., Asinex, Moscow, Ru, Tripos, Inc., St. Louis, Mo., ChemStar, Ltd, Moscow, RU, 3D Pharmaceuticals, Exton, Pa., Martek Biosciences, Columbia, Md., etc.).

Control reactions may be performed in combination with the libraries. Such optional control reactions are appropriate and increase the reliability of the screening. Accordingly, in a preferred embodiment, the methods of the invention include such a control reaction. The control reaction may be a negative control reaction that measures the transcription factor activity independent of a transcription modulator. The control reaction may also be a positive control reaction that measures transcription factor activity in view of a known transcription modulator.

By being able to screen multiple different transcription factors at the same time, not only is it possible to screen a large number of potential transcription modulators per day, it is also possible to screen any potential transcription modulator relative to a large number of different transcription factors. The ability to screen multiple different transcription factors at the same time thus greatly enhances the high throughput capabilities of this screening assay.

### d. Evaluation of Drug Efficacy

Given that certain disease states may be caused by an unusual level of transcription of one or more genes, drugs may be designed to either stimulate or inhibit transcription in order make gene expression of diseased cells approach the gene expression of normal cells. A rapid and effective method for monitoring gene expression is thus highly advantageous for evaluating the effectiveness of a drug's ability to alter the transcription of one or more genes. The effectiveness of a drug being delivered to a site of action as well as the drug's efficacy *in vivo* can thus be evaluated with the assistance of the methods of the present invention.

Also of great concern when developing new drugs is the side effects which the drugs might have. One approach for screening drug candidates for undesirable side effects would be to employ the present invention to monitor how gene expression is altered in response to the administration of a drug candidate. By understanding how a candidate affects gene expression, candidates likely to have undesirable side affects can be rapidly identified.

Because the biological importance of transcription factors, they are ideal drug targets. Traditional transcription factor screening assays only detect one transcription factor at a time. As a result, existing assays are tremendously in efficient for detecting how a drug effects different gene expression. However, with the assistance of the present invention, it is now possible to screen hundreds and even thousands of transcription factors in a short amount of time in order to monitor how a given drug affects the expression of wide range of genes. The present invention will thus dramatically facilitate the screening process of identifying new drugs, characterizing their mechanism of the action, and screening for adverse side effects based on the drug's impact on expression.

### 9. Determining Sequence Binding

### Requirements For Transcription Factors

In general, a further application of the present invention is the rapid and efficient determination of the DNA sequence binding requirements for a given transcription factor. By being able to efficiently isolate DNA probes from multiple DNA probe - transcription factor complexes, the present invention makes it feasible to identify which DNA sequences bind to transcription factors, quantify the amount of each DNA sequence isolated, and use that information to determine different DNA sequence binding requirements for a given transcription factor in a high throughput manner.

As a result of the completion of the human genome project, many new proteins will be discovered. Based on their primary structure, transcription factors for these proteins can be identified. However, the identification of DNA sequences to which these transcription factors will bind is a more difficult problem which the present invention helps to address.

When determining the DNA sequences to which transcription factors bind, there are several different questions that need to be answered. One question relates to the identification of an optimal binding sequence for the transcription factor. Another question relates to the identification of the minimal sequence required for binding. Yet another question which is related to the prior question, relates to the identification of a consensus sequence which is the minimum sequence required for binding. The present invention can be used to facilitate the answering of each of these questions.

The present invention enables one to rapidly determine a set of DNA sequences to which an activated transcription factor will bind and a set of DNA sequences to which an activated transcription factor will not bind. The amount isolated of each member in the set of DNA sequences to which an activated transcription factor binds may also be quantified.

Using this information, the minimum sequence required for the transcription factor to bind may be determined. Varying degrees of consensus sequences among the DNA sequences to which an activated transcription factor binds can also be determined. Meanwhile, by quantifying the amount of isolated DNA, how different base substitutions affect transcription factor binding can also be evaluated. This enables one to determine the sequences to which a given transcription factor binds most tightly.

Figure 4 illustrates a process whereby the minimum DNA sequence binding requirements for a given transcription factor can be rapidly determined. As illustrated, a sample containing an activated transcription factor is contacted with a library of probes. The probes in the library comprise a DNA sequence and a detectable marker.

Since, in this embodiment, the library of probes are used to determine the minimum DNA sequence binding requirements, the DNA sequences used in the library may be variations on a sequence which the transcription factor is known to bind to. Alternatively, the sequences used in the library may selected without knowledge of the binding specificity of the activated transcription factor.

If the DNA probes used to perform this method are known, a simple hybridization array having compliments to the DNA probes in the library may be employed, as described above. However, if a random library of DNA probes is employed, any isolated DNA probes can be characterized by existing position-fixed DNA array technology.

As a result of contacting the sample with the library of probes, complexes are formed between the activated transcription factor present in the sample and DNA probes in the library which have sequences that satisfy the sequence specificity of the DNA-binding domain of the activated transcription factor. The resulting probe - transcription factor complexes are preferably isolated by purification from agarose gel as described previously. After isolating the DNA probe - activated transcription factor complexes, those probes from the library which bind to transcription factors in the sample may be further isolated and characterized as discussed previously.

Since only those probes from the library which form a complex with an activated transcription factor will be isolated, identification of which probes are isolated serves to identify the range of sequences to which the activated transcription factor is capable of binding.

By constructing a consensus sequence based on the isolated probes, one is able to more precisely define the minimum binding requirements for the transcription factor. Furthermore, once a consensus sequence and a series of binding sequences are know, this information can be used to locate the occurrence of those sequences in 5' untranslation regions within a genome. This will allow researchers to identify which proteins may be regulated by the transcription factor. Genomes of different organisms may also be researched to identify proteins that may be functionally related.

Depending on the level of diversity of the library used, a further round of screening may be used to map the binding requirements of the transcription factor in greater detail. For example, when one or more binding sequences are identified, further experimentation may also be conducted to identify more binding sequences. This may involve creating a library of random mutations based on one or more DNA sequences shown to bind to the transcription factor in the prior screen. Binding of the mutants may be performed in order to identify other mutants to which the transcription factor binds. Multiple cycles of generating and screening mutant libraries may be conducted as is necessary and desirable.

Figure 5 illustrates a variation of the method described in regard to Figure 4 where an optimal sequence for binding is identified. As illustrated in Figure 5, the isolated DNA probes are quantified as well as identified. By monitoring how changes in the sequence affect the amount of each probe isolated, one is able to see how the different sequences compete for binding to the transcription factor. As a result, an optimal transcription factor binding sequence can be identified.

Depending on the level of diversity of the library used to perform the first screen, when one or more binding sequences are identified, further experimentation may also be conducted to identify a better binding sequence. This may involve creating a library of random mutations based on one or more DNA sequences shown to bind to the transcription factor in the prior screen. Quanitification of the binding of the mutants may be performed in order to identify a stronger binding mutant. Multiple cycles of generating and screening mutant libraries may be conducted as is necessary and desirable.

### 10. Determining Transcription Factor Expression and Activation

Prior to being activated, a transcription factor must be expressed. However, not all expressed transcription factors are activated. By determining whether multiple different transcription factors are being activated according to the present invention, in combination with determining whether the different transcription factors are being expressed, the present invention provides a rapid and efficient method for monitoring how different transcription factor expression and transcription factor activation change.

One application of this method relates to the diagnosis of disease states. By being able to track both changes in transcription factor expression and activation, more precise diagnosis of the cause of genetically related diseases may be discovered.

A further application of this method relates to the evaluation of how different agents or conditions affect both transcription factor expression and activation. For example, different agents can be screened for their ability to inhibit and/or activate the expression of certain proteins or impact different disease states. By knowing how the agent affects both transcription factor expression and activation, one is able to identify the mode of action of the agent. By being able to screen multiple transcription factors at the same time, one is further able to screen those agents for otherwise unforeseen adverse affects on the genetic level.

### 11. Method For Detecting Interactions Between Transcription Factors and Other Proteins

Yet a further application is the rapid and efficient determination of interactions that occur between a given transcription factor and other proteins. In one particular instance, the other proteins are other transcription factors. It is noted that the method may be more broadly applied to detecting the interaction between a given protein and other proteins.

Transcription factors generally have at least two domains: a DNA binding domain which binds to promoter and enhancer modules; and an activation domain that allows for interaction between transcription factors and/or with RNA polymerase II. The role played by interactions between transcription factors is not yet well understood but is believed to be significant. The role played by interactions between transcription factors and other proteins is also not well understood.

One of the challenges associated with elucidating the roles that interactions between transcription factors and other proteins including other transcription factors play is the identification of the different complexes that transcription factors form. For example, a mammal is believed to express over 1000 different transcription factors. These different transcription factors are expressed by different cells and under different conditions. Determining which transcription factors form complexes with other proteins, including other transcription factors under different conditions can be very difficult. A need thus exists for a high throughput method for detecting transcription factor - transcription factor and other transcription factor - protein complexes.

Figure 19 illustrates an embodiment of a method provided according to the present invention for detecting transcription factor - transcription factor complexes. A biological sample to be analyzed is typically derived from a sample of cells and is preferably a nuclear extract of the cell sample. The sample contains an unknown mixture of transcription factors.

Optionally, the biological sample may be contacted with a library of probes. These probes may be used later on as detectable markers or to identify the presence of a given transciption factor. Each transcription factor probe in the library comprises a DNA sequence that is capable of binding to an activated transcription factor, referred to herein as the probe's recognition sequence. At least the recognition sequence varies within the library of probes such that the probes are capable of binding to a plurality of different activated transcription factors. Due to the high level binding specificity of the transcription factors, each transcription factor probe has binding specificity for a single transcription factor or family of transcription factors. The probes in the library are preferably double stranded. One of the strands is preferably biotin labeled at the 5' end to facilitate detection.

Because the method may be directed toward identifying known transcription factors, it is practical to use longer recognition sequences in the probes in the library as compared to what would be practical if a random library were used to identify previously unknown transcription factors. As a result, at least 1%, 2%, 3%, 5%,10%, 20%, 30%, 50% or more of the probes in the library may have recognition sequences greater than 35, 40, 45 or more base pairs in length. By using longer recognition sequences, the probes have greater binding specificity. In addition, the probes have greater binding efficiency to the transcription factors which improves the yield of probe - transcription factor complexes isolated. As a result, the method of the present invention provides a high level of sensitivity for isolating transcription factor - transcription factor complexes in combination with a high signal to noise ratio.

As a result of contacting the sample with the library of transcription factor probes, complexes are formed between activated transcription factors present in the sample and transcription factor probes in the library which have sequences that match the sequence specificity of the DNA-binding domains of the activated transcription factors.

Some of the transcription factors in the sample may form complexes between one or more different transcription factors. In order to detect and determine the composition of these transcription factor - transcription factor complexes, complexes which comprise a single type of transcription factor may be isolated. Isolation of a single type of transcription factor may be performed by contacting the transcription factor complexes with an antibody or other agent that can specifically bind to a particular type of transcription factor. The agent that specifically binds to the particular type is then used to separate transcription factor complexes comprising the particular type of transcription factor. Alternatively, the agent that specifically binds to the particular type of transcription factor may be immobilized on a solid support which thus serves to immobilize transcription factor complexes comprising the particular type of transcription factor.

Since the agent used to isolate the transcription factor complexes is specific for a single type of transcription factor, no other transcription factors should be isolated unless the transcription factor is part of a complex comprising the particular type of transcription factor upon which the isolation is based.

Identification of the different types of transcription factors present in the pool of isolated complexes may be determined either by identifying which transcription factors are present, or by isolating the transcription factor probes bound to the transcription factors and characterizing them. Both alternatives are shown in Figure 19.

On the left side of Figure 19, the transcription factor probes contained among the pool of isolated complexes are characterized. The transcription factor probes may be characterized by any methodology available for characterizing DNA probes. In accordance with the invention, the isolated transcription factor probes are characterized using an array of hybridization probes such as the array that has already been described. In another instance, the isolated transcription factor probes are characterized using an array of proteins that selectively bind to the different isolated transcription factor probes. These proteins may be antibodies against recognition sequences of the probes or proteins which recognize the recognition sequences (e.g., transcription factors) for the isolated transcription factor probes.

Detection of transcription factor probes by an array is advantageous because it facilitates the detection of multiple different transcription factor probes in a single assay. For example, 5, 10, 20, 50 or more different transcription factor probes may be detected in the same assay.

Since only those probes from the library which form a complex with an activated transcription factor will be isolated, identification of which probes are isolated serves to identify which activated transcription factors are present. Given that the isolation process is designed to only isolate a single type of transcription factor, the presence of probes for other types of transcription factors indicates that these other types of transcription factors formed complexes with the type of transcription factor upon which the isolation was based.

On the right side of Figure 19, an alternative process for characterizing the transcription factors contained within the pool of isolated complexes is illustrated. As shown, transcription factors are characterized. The transcription factors may be characterized by any methodology available for characterizing proteins. In one instance, the isolated transcription factors are characterized using an array of hybridization probes which comprise recognition sequences for the different transcription factors that might be isolated. In another instance, the isolated transcription factors are characterized using an array of agents, such as antibodies, which selectively bind to different transcription factors that might be isolated.

Detection of transcription factors by an array is advantageous because it facilitates the detection of multiple different transcription factors in a single assay. For example, 5, 10, 20, 50 or more different transcription factors may be detected in the same assay.

By performing the method illustrated in Figure 19, it is possible to isolate and then optionally characterize all transcription factors that form a complex with a given type of transcription factor. As noted previously, a mammalian cell can express more than 1000 different types of transcription factors. Accordingly, the above described process allows one to simultaneous screen for transcription factor - transcription factor complexes between a given transcription factor and the different types of transcription factors that may be present in the cell. The above described process thus greatly reduces the amount of work that otherwise would need to be performed in order to monitor all these potential transcription factor - transcription factor complexes.

It is noted that the above method is described with regard to isolating transcription factor complexes based on a single type of transcription factor at a time. In order to determine transcription factor - transcription factor complexes involving a different type of transcription factor, the method may be repeated where an agent is employed for isolating a second different type of transcription factor. This process may be repeated for however many transcription factors as one might wish.

It is noted that isolating transcription factor complexes may also be based on isolating more than one type of transcription factor at a time. However, if isolation is performed based on two or more transcription factors at a time, there will be inherent ambiguity regarding which types of transcription factors are involved in a given complex.

A method is described for detecting transcription factor - protein complexes. This method may be used for detecting complexes between different transcription factors and/or for detecting complexes between a non-transcription factor protein and a transcription factor. It may be used more generally to detect complexes between two or more non-transcription factor proteins.

As noted above, the biological sample to be analyzed is typically derived from a sample of cells and is preferably a nuclear extract of the cell sample. Some of the transcription factors in the sample may form complexes with other proteins which may or may not be transcription factors. In order to detect and determine the composition of these transcription factor - protein complexes, complexes which comprise a single type of protein may be isolated.

Isolation of a single type of protein (whether or not a transcription factor) may be performed by contacting the protein with an antibody or other agent that can specifically bind to the particular type of protein. The agent that specifically binds to the particular type of protein is then used to separate complexes comprising the particular type of protein. Alternatively, the agent that specifically binds to the particular type of protein may be immobilized on a solid support which thus serves to immobilize complexes comprising the particular type of protein.

Since the agent used to isolate the protein complexes is specific for a single type of protein (e.g., a particular type of transcription factor or a particular type of protein other than a transcription factor), no other proteins + should be isolated unless the protein is part of a complex comprising the particular protein upon which the isolation is based.

Identification of proteins present in the pool of isolated complexes may be performed by any method available for detecting proteins. For example, proteins contained within the pool of isolated complexes may be characterized using an array of agents, such as antibodies, which selectively bind to different proteins that might have been isolated. The agents in the array may be directed toward transcription factors, non-transcription factor proteins, and combinations thereof.

Detection of proteins by an array is advantageous because it facilitates the detection of multiple different proteins in a single assay. For example, 5, 10, 20, 50 or more different proteins may be detected in the same assay.

According to this method, it is possible to isolate and then optionally characterize all transcription factors that form a complex with a given type of protein upon which an isolation is based. As noted previously, a mammalian cell can express more than 1000 different types of transcription factors. Accordingly, the above described process allows one to simultaneous screen for transcription factor - protein complexes between a given protein and the different types of transcription factors. Alternatively, one may simultaneously screen for transcription factor - protein complexes between a given transcription factor and multiple different types of proteins that may also be present in a cell. The parallel screening allowed by the present invention greatly reduces the amount of work that otherwise would need to be performed in order to monitor all these potential transcription factor - protein complexes.

### 12. Kits For Use In The Present Invention

A wide variety of kits may be designed for use with the present invention. Various examples of these kits have already been described and additional kits are further described herein.

A kit in accordance with the invention includes a library of transcription factor probes comprising recognition sequences for transcription factors. The kit further includes an array of hybridization probes where each probe comprises a sequence that is complementary to at least a portion of the recognition sequences on the probes in the library. The probes in the array of hybridization probes comprise 2, 3, 4, or more copies of a sequence that is complementary to at least a portion of the recognition sequences on the probes in the library.

A kit is described which includes a first library of transcription factor probes comprising recognition sequences for transcription factors, each probe in the first library further comprising a first detectable marker. The kit further includes a second library of transcription factor probes comprising the same recognition sequences for transcription factors as the first library, each probe in the second library further comprising a second detectable marker that is different than the first detectable marker.

A kit is described which includes a library of transcription factor probes comprising recognition sequences for transcription factors in combination with instructions for using agarose gel to isolate transcription factor probe - transcription factor complexes.

A kit is described which includes an array of hybridization probes where each probe comprises a sequence that is complementary to at least a portion of recognition sequences of transcription factors. The kit further includes instructions for using agarose gel to isolate transcription factor probe - transcription factor complexes.

A kit is described which includes a hybridization array according to the present invention, hybridization buffer, detection wash buffer, and detection equilibrate buffer.

With regard to any of the kit embodiments, it is noted that the libraries of transcription factor probes and hybridization arrays may be varied as has been described herein.

### 13. Array Detection of Different Activated Transcription Factors

This section describes experimental results achieved by employing a library of probes according to the present invention to simultaneously screen samples of cells for 54 different activated transcription factors.

Figure 6 provides the sequences for the probes used to form the transcription factor probe library used in this experiment and the experiments described in Sections 14-20 herein. It is noted that the probes used were doubled stranded, Figure 6 only showing the strand with biotin labeled at the 5' end. The strands not shown are the complements to the sequences shown in Figure 6.

Figure 6 also shows the hybridization probes used in the hybridization probe array used in this experiment. Figure 7 shows the layout of the array of hybridization probes employed in the experiments described herein.

As can be seen in Figure 7, each hybridization probe was placed in multiple different regions, in this case, 4 separate regions. It is noted that 2, 3, 4 or more multiple separate regions may be employed. Alternatively, only a single region may also be employed. As can also be seen in Figure 7, the concentration of hybridization probe was varied in the different regions. This serves both as an internal control as well as a mechanism for quantifying the amount of immobilized probes.

Biotinylated oligonucleotides which are used as controls were positioned in the regions of Row O and Column 17. These oligonucleotides also serve as a legend for the array, allowing a person to identify positions of rows and columns in the array.

The transcription factor probe library described in Figure 6 was contacted with the array of hybridization probes described in regard to Figures 6 and 7. In this instance, unlike the experiments to be described herein, no intermediate isolation of transcription factor probe - transcription factor complexes was performed.

Figure 8 is an image of the resulting array. As can be seen, all of the regions contain immobilized transcription factor probes. As can also be seen, the regions with higher concentrations of the same hybridization probe appear brighter (e.g., A1 and A2 vs. B1 and B2).

### 14. Array Detection of Selected Activated Transcription Factors

Specific transcription factor probes and combinations of transcription factor probes were also contacted with a nuclear extract from HeLa cells and probes from any transcription factor probe - transcription factor complexes that formed being isolated. The transcription factor probes and combinations of transcription factor probes used were Brn3, c-Myb, Smad3/4 individually and the combination of Brn3, c-Myb, and Smad3/4.

Figures 9A-9D are images of the resulting arrays. As can be seen, only the hybridization probe regions for Brn3, c-Myb, and Smad3/4 appear to possess immobilized transcription factor probes in Figures 9A-9C respectively. Meanwhile, Figure 9D shows immobilized transcription factor probes in the Brn3, c-Myb, and Smad3/4 hybridization probe regions. The ratios of the spot densities among Brn3, c-Myb, and Smad3/4 has been found to be very similar to what is observed in arrays with single probe detection.

As can also be seen, the higher concentration regions appear consistently darker than the lower concentration regions. The use of regions with different concentrations of hybridization probes allows different concentrations of transcription factor probes to be isolated. If the higher concentration region is saturated, the lower concentration one can be used for evaluation.

### 15. Array Detection of Activated Transcription Factorsin HeLa Cells

The entire library of probes described in regard to Figure 6 was also contacted with a nuclear extract from HeLa cells and probes from any transcription factor probe - transcription factor complexes that formed being isolated.

Figure 10B is an image of the resulting array. As a control, the entire library of probes described in regard to Figure 6 was also contacted with a control sample that did not contain any transcription factors. Probes from any transcription factor probe - transcription factor complexes that formed were isolated. Since no transcription factors are present in the control sample, it is expected that no complexes are formed and hence no probes are isolated. Figure 10A is an image of the resulting array.

As can be seen in Figure 10A and as would be expected, no transcription factor probes are immobilized in the array for the control sample. Meanwhile, as can be seen in Figure 10B, a myriad of transcription factor probes are immobilized in the array for the HeLa cell sample.

### 16. Comparison Between Activated Transcription Factors in HeLa Cells and PMA-activated HeLa Cells

Figure 11A is an image of the array resulting from the entire library of probes described in regard to Figure 6 being contacted with a nuclear extract from HeLa cells and any probes from any transcription factor probe - transcription factor complexes that formed being isolated.

In comparison, Figure 11B is an image of the array resulting from the entire library of probes described in regard to Figure 6 being contacted with a nuclear extract from PMA-treated HeLa cells and probes from any transcription factor probe - transcription factor complexes that formed being isolated.

As can be seen by comparing Figures 11A and 11B, a number of transcription factors including Ets and NF-E1 can be seen to have been activated at higher levels in the PMA-treated HeLa cells.

The arrays shown in Figures 11A and 11B were imaged using a FluorChem imager (from Alpha Innotech Corp) in order to quantify the intensity of the spots appearing in the different regions of the array. Figures 12A and 12B provide tables of the signal intensity for the arrays shown in Figures 11A and 11B. Figure 12C provides the ratio between the intensities shown in Figures 12A and 12B.

As can be seen by comparing the data shown in Figures 12A and 12B, the signal intensities for the regions associated with transcription factors Egr (C5, C6, D5, D6); Ets (E9, E10, F9, F10); NF-E1 (G4, G5, H4, H5); and Smad3/4 (I15, I6, J15, J16) are more intense. Meanwhile, Ets (E9, E10, F9, F10) and Smad3/4 (I15, I16, J15, J16) show no difference in density.

The results of the experiment described in regard to Figures 11A, 11B, and 12A-12C was confirmed by performing a standard gel shift assay using Ets and NF-E1 probes. Specifically, nuclear extracts of HeLa and PMA-treated HeLa cells were incubated with Ets and NF-E1 probes respectively. As can be seen in Figure 13, bands corresponding to Ets appear for both PMA-treated and untreated HeLa cells. By contrast, a band corresponding to NF-E1 was not present in untreated HeLa cells but appeared in PMA-treated HeLa cells.

### 17. Comparison Between Activated Transcription Factors in A431 Cellsand PMA-activated A431 Cells

The entire library of probes described in regard to Figure 6 was also contacted with a nuclear extract untreated A431 cells and PMA-treated A431 cells. Figure 14A is an image of the array for untreated A431 cells and Figure 14B is an image of the array for PMA-treated A431 cells. As can be seen by comparing Figures 14A and 14B, transcription factors NF-E1 and NF-kB were found to be activated by PMA in A431 cells.

The results of the experiment described in regard to Figures 14A and 14B was confirmed by performing a standard gel shift assay using Ets, NF-E1, and NF-kB probes. Specifically, nuclear extracts of A431 and PMA-treated A431 cells were incubated with Ets, NF-E1, and NF-kB probes respectively. As can be seen in Figure 15, bands corresponding to Ets appear for both PMA-treated and untreated A431 cells. By contrast, bands corresponding to NF-E1 and NF-kB were not present in untreated A431 cells but appeared in PMA-treated A431 cells.

### 18. Comparison Between Activated Transcription Factors in Jurkat Cells and PMA-activated Jurkat Cells

The entire library of probes described in regard to Figure 6 was also contacted with a nuclear extract untreated Jurkat cells and PMA-treated Jurkat cells. Figure 16A is an image of the array for untreated Jurkat cells and Figure 16B is an image of the array for PMA-treated Jurkat cells. As can be seen by comparing Figures 16A and 16B, transcription factor AP1 was found to be activated by PMA. Interestingly, NF-E1 was not induced by PMA, showing that NF-E1 induction by PMA is cell line dependent.

### 19. Comparison Of Activated Transcription Factors Between Multiple Cell Lines

The entire library of probes described in regard to Figure 6 was also contacted with nuclear extracts for HeLa, A431, Jurkat, K-562, and Y79 cells in order to compare the activated transcription factors present in these different cell lines. Figures 17A-17E show the resulting arrays for HeLa, A431, Jurkat, K-562, and Y79 cells respectively. As can be seen, the mixture of transcription factors that are activated varies for the different cells.

### 20. Gel Shift Analyses Of Different Transcription Factors

Gel shift analyses were performed for multiple different transcription factors in order evaluate the sensitivity of gel shift analysis for detecting different transcription factors. Specifically, a nuclear extract of HeLa cells was incubated with transcription factor probes for c-Myb, Ets, Smad3/4, Brn3 and NF-E2. Figure 18A is an image of a polyacrylamide gel and Figure 18B is an image of an agarose gel. As can be seen in both figures, a band corresponding to probe - transcription factor complexes is effectively separated. As can also be seen, c-Myb, Ets, and Smad3/4 can be detected. However, Brn3 and NF-E2 are difficult to detect. By contrast, c-Myb, Ets, Smad3/4, Brn3 and NF-E2 can all be detected in the array shown in Figure 10B.

It will be apparent to those skilled in the art that various modifications and variations can be made in the kits and methods of the present invention without departing from the scope of the invention as defined by the claims.

## Claims

1. A method for identifying which of a plurality of different activated transcription factors are present in a biological sample, the method comprising:
contacting the biological sample with a library of double stranded DNA probes under conditions where DNA probe - transcription factor complexes are formed between the DNA probes in the library and activated transcription factors present in the biological sample, the DNA probes each comprising a known transcription factor recognition sequence varying within the library such that different DNA probes bind different activated transcription factors, wherein a different DNA probe is provided for each different activated transcription factor in the plurality of activated transcription factors;
isolating the DNA probes that have bound to the activated transcription factors and formed the DNA probe - transcription factor complexes; and
contacting the isolated DNA probes with an array of immobilized hybridization probes under conditions suitable for hybridization of at least one of the strands of the different double stranded DNA probes to the hybridization probes in the array, wherein a different hybridization probe is provided for each different double stranded DNA probe, and wherein identification of the DNA probes bound to the array identifies which of the plurality of different activated transcription factors are present in the biological sample.

2. A method according to claim 1, in which one strand of the double stranded DNA probes comprises a detectable marker, the method further including using the detectable marker to identify which of the isolated DNA probes hybridize to the array.

3. A method according to claim 2, in which the detectable marker, preferably biotin, is at a 5' end of the strand.

4. A method according to claim 1, 2 or 3, in which each of the immobilized hybridization probes comprises a transcription factor probe binding region comprising at least two copies of a complement to a portion of the transcription factor recognition sequence on one of the DNA probes.

5. A method for screening for transcription factor modulators, the method comprising:
forming a plurality of test samples by contacting samples of cells with different agents; and
for each test sample, identifying which of a plurality of different activated transcription factors are present by
contacting the test sample with a library of double stranded DNA probes under conditions where DNA probe - transcription factor complexes are formed between the DNA probes in the library and activated transcription factors present in the test sample, the DNA probes each comprising a known transcription factor recognition sequence varying within the library such that different DNA probes bind different activated transcription factors, wherein a different DNA probe is provided for each different activated transcription factor in the plurality of activated transcription factors,
isolating the DNA probes that have bound to the activated transcription factors and formed the DNA probe -transcription factor complexes, and
contacting the isolated DNA probes with an array of immobilized hybridization probes under conditions suitable for hybridization of at least one of the strands of the different double stranded DNA probes to the hybridization probes in the array, wherein a different hybridization probe is provided for each different double stranded DNA probe, and wherein identification of the DNA probes bound to the array identifies which of the plurality of different activated transcription factors are present in the test sample; and
comparing the activated transcription factors present in the different test samples.

6. A method according to claim 5, in which each of the immobilized hybridization probes comprises a transcription factor probe binding region comprising at least two copies of a complement to a portion of the transcription factor recognition sequence on one of the DNA probes.

7. A method according to claim 5 or claim 6, in which one strand of each of the double stranded DNA probes comprises a detectable marker, preferably biotin, preferably at a 5' end of the strand.

8. A method according to any preceding claim, in which the library comprises double stranded DNA probes having transcription factor recognition sequences between 20 and 40 base pairs in length, preferably between 25 and 35 base pairs in length.

9. A method according to any preceding claim, in which the library of double stranded DNA probes comprises at least 5, preferably at least 10, preferably at least 20, more preferably at least 50 different transcription factor recognition sequences.

10. A method according to any preceding claim, in which the library of double stranded DNA probes comprises transcription factor recognition sequences for at least 2, preferably at least 5, more preferably at least 10 different types of cells.

11. A method according to any preceding claim, in which the library of double stranded DNA probes comprises transcription factor recognition sequences for malignant, benign, and normal cell types.

12. A method according to any preceding claim, in which the biological sample is a nuclear extract.

13. A method according to any preceding claim, in which isolating the DNA probes that have bound to the activated transcription factors and formed the DNA probe - transcription factor complexes comprises binding the DNA probe - transcription factor complexes to a nitrocellulose filter.

14. A method according to any one of claims 1 to 12, in which isolating the DNA probes that have bound to the activated transcription factors and formed the DNA probe-transcription factor complexes comprises separating the DNA probe - transcription factor complexes from DNA probes not bound to activated transcription factors using an agarose gel.

15. A kit for use in identifying which of a plurality of different activated transcription factors are present in a biological sample by identifying which members of a library of double stranded DNA probes form DNA probe - transcription factor complexes with activated transcription factors present in the biological sample, the kit comprising:
the library of double stranded DNA probes, the DNA probes each comprising a known transcription factor recognition sequence varying within the library such that different DNA probes bind different activated transcription factors, wherein a different DNA probe is provided for each different activated transcription factor in the plurality of activated transcription factors; and
a hybridization array comprising:
a substrate; and
a plurality of hybridization probes immobilized on a surface of the substrate such that different hybridization probes are positioned in different defined regions on the surface, wherein a different hybridization probe is provided for each different double stranded DNA probe, and wherein each different hybridization probe comprises a transcription factor probe binding region comprising at least two copies of a complement to a portion of the transcription factor recognition sequence on one of the DNA probes.

16. A kit according to claim 15, in which the array further comprises an internal standard, preferably biotinylated DNA.

17. A kit according to claim 15 or 16, in which the array further comprises biotinylated DNA which is positioned on the array so as to define a grid pattern of the array.

18. A kit according to any of claims 15 to 17, in which the different hybridization probes on the array are collectively capable of immobilizing at least 5, preferably at least 10, preferably at least 20, more preferably at least 50 different DNA probes to the array.

19. A kit according to any of claims 15 to 18, in which the different hybridization probes on the array are collectively capable of immobilizing DNA probes for at least 2, preferably at least 5, more preferably at least 10 different types of cells to the array.

20. A kit according to any of claims 15 to 19, in which each different hybridization probe is positioned in two or more different regions.

21. A kit according to claim 20, in which the concentration of each different hybridization probe is varied in the two or more different regions.

## Patentansprüche

1. Verfahren zur Bestimmung, welche aktivierten Transkriptionsfaktoren einer Vielzahl von unterschiedlichen aktivierten Transkriptionsfaktoren in einer biologischen Probe vorliegen, wobei das Verfahren die folgenden Schritte umfasst:
Inkontaktbringung der biologischen Probe mit einer Sammlung doppelsträngiger DNA-Sonden unter Bedingungen, unter welchen DNA-Sonde-Transkriptionsfaktorkomplexe zwischen den DNA-Sonden in der Sammlung und den in der biologischen Probe vorliegenden aktivierten Transkriptionsfaktoren gebildet werden, wobei jede DNA-Sonde eine bekannte Transkriptionsfaktor-Erkennungssequenz aufweist, welche innerhalb der Sammlung derart variiert, dass verschiedene DNA-Sonden unterschiedliche aktivierte Transkriptionsfaktoren binden, wobei für jeden unterschiedlichen aktivierten Transkriptionsfaktor in der Vielzahl von aktivierten Transkriptionsfaktoren eine unterschiedliche DNA-Sonde bereitgestellt ist;
Isolierung der DNA-Sonden, welche sich an die aktivierten Transkriptionsfaktoren gebunden haben und die DNA-Sonde-Transkriptionsfaktorkomplexe gebildet haben; und
Inkontaktbringung der isolierten DNA-Sonden mit einer Anordnung immobilisierter Hybridisierungssonden unter Bedingungen, welche für eine Hybridisierung zumindest eines Strangs der verschiedenen doppelsträngigen DNA-Sonden an die Hybridisierungssonden in der Anordnung geeignet sind, wobei für jede unterschiedliche doppelsträngige DNA-Sonde eine unterschiedliche Hybridisierungssonde bereitgestellt ist, und wobei eine Bestimmung der an die Anordnung gebundenen DNA-Sonden bestimmt, welche aktivierten Transkriptionsfaktoren der Vielzahl von unterschiedlichen aktivierten Transkriptionsfaktoren in der biologischen Probe vorhanden sind.

2. Verfahren nach Anspruch 1, in welchem ein Strang der doppelsträngigen DNA-Sonden einen nachweisbaren Marker aufweist, wobei das Verfahren des Weiteren den Schritt der Verwendung des nachweisbaren Markers einschließt, um zu bestimmen, welche der isolierten DNA-Sonden an die Anordnung hybridisieren.

3. Verfahren nach Anspruch 2, in welchem der nachweisbare Marker, vorzugsweise Biotin, an einem 5'-Ende des Strangs angeordnet ist.

4. Verfahren nach Anspruch 1, 2 oder 3, in welchem jede der immobilisierten Hybridisierungssonden einen Transkriptionsfaktorsonde-Bindungsbereich aufweist, welcher mindestens zwei Exemplare eines komplementären Gegenstücks zu einem Abschnitt der Transkriptionsfaktor-Erkennungssequenz auf einer der DNA-Sonden umfasst.

5. Verfahren für eine Untersuchung auf Transkriptionsfaktor-Modulatoren, wobei das Verfahren die folgenden Schritte umfasst:
Bildung einer Vielzahl von Testproben durch Inkontaktbringung von Zellproben mit unterschiedlichen Stoffen; und
Bestimmung für jede Testprobe, welche unterschiedlichen aktivierten Transkriptionsfaktoren der Vielzahl von aktivierten Transkriptionsfaktoren vorhanden sind, indem
die Testprobe mit einer Sammlung doppelsträngiger DNA-Sonden unter Bedingungen in Kontakt gebracht wird, unter denen DNA-Sonde-Transkriptionsfaktorkomplexe zwischen den DNA-Sonden in der Sammlung und den in der Testprobe vorhandenen aktivierten Transkriptionsfaktoren gebildet werden, wobei jede DNA-Sonde eine bekannte Transkriptionsfaktor-Erkennungssequenz aufweist, welche innerhalb der Sammlung derart variiert, dass unterschiedliche DNA-Sonden unterschiedliche aktivierte Transkriptionsfaktoren binden, wobei für jeden unterschiedlichen aktivierten Transkriptionsfaktor in der Vielzahl von aktivierten Transkriptionsfaktoren eine unterschiedliche DNA-Sonde vorgesehen ist;
Isolierung der DNA-Sonden, welche sich an die aktivierten Transkriptionsfaktoren gebunden haben und die DNA-Sonde-Transkriptionsfaktorkomplexe gebildet haben; und
Inkontaktbringung der isolierten DNA-Sonden mit einer Ansammlung immobilisierter Hybridisierungssonden unter Bedingungen, welche für eine Hybridisierung zumindest eines Strangs der unterschiedlichen doppelsträngigen DNA-Sonden an die Hybridisierungssonden in der Anordnung geeignet sind, wobei für jede unterschiedliche doppelsträngige DNA-Sonde eine unterschiedliche Hybridisierungssonde vorgesehen ist, und wobei die Bestimmung der an die Anordnung gebundenen DNA-Sonden bestimmt, welche aktivierten Transkriptionsfaktoren der Vielzahl von unterschiedlichen aktivierten Transkriptionsfaktoren in der Testprobe vorhanden sind; und
Vergleich der aktivierten Transkriptionsfaktoren, welche in den unterschiedlichen Testproben vorhanden sind.

6. Verfahren nach Anspruch 5, in welchem jede der immobilisierten Hybridisierungssonden einen Transkriptionsfaktorsonde-Bindungsbereich aufweist, welcher mindestens zwei Exemplare eines komplementären Gegenstücks zu einem Abschnitt der Transkriptionsfaktor-Erkennungssequenz auf einer der DNA-Sonden umfasst.

7. Verfahren nach Anspruch 5 oder Anspruch 6, in welchem ein Strang einer jeden doppelsträngigen DNA-Sonde einen nachweisbaren Marker, vorzugsweise Biotin, vorzugsweise an einem 5'-Ende des Strangs aufweist.

8. Verfahren nach einem der vorhergehenden Ansprüche, in welchem die Sammlung von doppelsträngigen DNA-Sonden Transkriptionsfaktor-Erkennungssequenzen mit einer Länge von zwischen 20 und 40 Basenpaaren, vorzugsweise zwischen 25 und 35 Basenpaaren, umfasst.

9. Verfahren nach einem der vorhergehenden Ansprüche, in welchem die Sammlung doppelsträngiger DNA-Sonden mindestens 5, vorzugsweise mindestens 10, vorzugsweise mindestens 20, noch bevorzugter mindestens 50 unterschiedliche TranskriptionsfaktorErkennungssequenzen aufweist.

10. Verfahren nach einem der vorhergehenden Ansprüche, in welchem die Sammlung doppelsträngiger DNA-Sonden Transkriptionsfaktor-Erkennungssequenzen für mindestens 2, vorzugsweise mindestens 5, noch bevorzugter mindestens 10 unterschiedliche Zelltypen aufweist.

11. Verfahren nach einem der vorhergehenden Ansprüche, in welchem die Sammlung von doppelsträngigen DNA-Sonden Transkriptionsfaktor-Erkennungssequenzen für bösartige, gutartige und normale Zelltypen umfasst.

12. Verfahren nach einem der vorhergehenden Ansprüche, in welchem die biologische Probe ein nukleärer Extrakt ist.

13. Verfahren nach einem der vorhergehenden Ansprüche, in welchem die Isolierung der DNA-Sonden, welche sich an die aktivierten Transkriptionsfaktoren gebunden haben und die DNA-Sonde-Transkriptionsfaktorkomplexe gebildet haben, die Bindung der DNA-Son-de-Transkriptionsfaktorkomplexe an einen Nitrozellulose-Filter umfassen.

14. Verfahren nach einem der Ansprüche 1 bis 12, in welchem die Isolierung der DNA-Sonden, welche sich an die aktivierten Transkriptionsfaktoren gebunden haben und die DNA-Sonde-Transkriptionsfaktorkomplexe gebildet haben, die Trennung der DNA-Sonde-Trans-kriptionsfaktorkomplexe von DNA-Sonden, die sich nicht an aktivierte Transkriptionsfaktoren gebunden haben, unter Verwendung eines Agarose-Gels umfasst.

15. Ausrüstung zur Verwendung bei der Bestimmung, welche aktivierten Transkriptionsfaktoren einer Vielzahl von unterschiedlichen aktivierten Transkriptionsfaktoren in einer biologischen Probe vorliegen, indem bestimmt wird, welche Angehörigen einer Sammlung von doppelsträngigen DNA-Sonden DNA-Sonde-Transkriptionsfaktorkomplexe mit den in der biologischen Probe vorliegenden aktivierten Transkriptionsfaktoren bilden, wobei die Ausrüstung Folgendes umfasst:
die Sammlung doppelsträngiger DNA-Sonden, wobei jede DNA-Sonde eine bekannte Transkriptionsfaktor-Erkennungssequenz aufweist, welche innerhalb der Sammlung derart variiert, dass unterschiedliche DNA-Sonden unterschiedliche aktivierte Transkriptionsfaktoren binden, wobei für jeden unteschiedlichen aktivierten Transkriptionsfaktor in der Vielzahl von aktivierten Transkriptionsfaktoren eine unterschiedliche DNA-Sonde vorgesehen ist; und
eine Hybridisierungs-Anordnung, welche Folgendes aufweist:
ein Substrat; und
eine Vielzahl von Hybridisierungssonden, welche auf einer Oberfläche des Substrats derart immobilisiert sind, dass unterschiedliche Hybridisierungssonden in unterschiedlichen definierten Bereichen der Oberfläche angeordnet sind, wobei für jede doppelsträngige DNA-Sonde eine unterschiedliche Hybridisierungssonde vorgesehen ist, und wobei jede unterschiedliche Hybridisierungssonde ein Transkriptionsfaktorsonde-Bindungsbereich vorgesehen ist, welcher mindestens zwei Exemplare eines komplementären Gegenstücks zu einem Abschnitt der Transkriptionsfaktor-Erkennungssequenz auf einer der DNA-Sonden aufweist.

16. Ausrüstung nach Anspruch 15, in welcher die Anordnung des Weiteren einen internen Standard, vorzugsweise biotinilierte DNA aufweist.

17. Ausrüstung nach Anspruch 15 oder 16, in welcher die Anordnung des Weiteren biotinilierte DNA aufweist, welche derart auf der Ansammlung angeordnet ist, dass sie ein Gittermuster der Anordnung definiert.

18. Ausrüstung nach einem der Ansprüche 15 bis 17, in welcher die unterschiedlichen Hybridisierungssonden auf der Ansammlung kollektiv in der Lage sind, mindestens 5, vorzugsweise mindestens 10, vorzugsweise mindestens 20, noch bevorzugter mindestens 50 unterschiedliche DNA-Sonden auf der Anordnung zu immobilisieren.

19. Ausrüstung nach einem der Ansprüche 15 bis 18, in welcher die unterschiedlichen Hybridisierungssonden auf der Anordnung kollektiv in der Lage sind, DNA-Sonden für mindestens 2, vorzugsweise für mindestens 5, noch bevorzugter für mindestens 10 unterschiedliche Zelltypen in der Anordnung zu immobilisieren.

20. Ausrüstung nach einem der Ansprüche 15 bis 19, in welcher jede verschiedene Hybridisierungssonde in zwei oder mehreren unterschiedlichen Bereichen angeordnet ist.

21. Ausrüstung nach Anspruch 20, in welcher die Konzentration einer jeden unterschiedlichen Hybridisierungssonde in den zwei oder mehreren unterschiedlichen Bereichen variiert.

## Revendications

1. Procédé pour identifier, parmi une pluralité de facteurs de transcription activés différents, ceux qui sont présents dans un échantillon biologique, le procédé comprenant :
la mise en contact de l'échantillon biologique avec une banque de sondes d'ADN double brin dans des conditions où des complexes sonde d'ADN-facteur de transcription sont formés entre les sondes d'ADN dans la banque et les facteurs de transcription activés présents dans l'échantillon biologique, les sondes d'ADN comprenant chacune une séquence de reconnaissance de facteur de transcription connue qui est variable au sein de la banque de sorte que des sondes d'ADN différentes se lient à des facteurs de transcription activés différents, où une sonde d'ADN différente est mise à disposition pour chaque facteur de transcription activé différent dans la pluralité de facteurs de transcription activés ;
l'isolement des sondes d'ADN qui se sont liées aux facteurs de transcription activés et ont formé les complexes sonde d'ADN-facteur de transcription ; et
la mise en contact des sondes d'ADN isolées avec un réseau de sondes d'hybridation immobilisées dans des conditions appropriées pour permettre une hybridation d'au moins un des brins des sondes d'ADN double brin différentes aux sondes d'hybridation dans le réseau, où une sonde d'hybridation différente est mise à disposition pour chaque sonde d'ADN double brin différente, et où l'identification des sondes d'ADN liées au réseau permet d'identifier, parmi la pluralité de facteurs de transcription activés différents, ceux qui sont présents dans l'échantillon biologique.

2. Procédé selon la revendication 1, dans lequel un brin des sondes d'ADN double brin comprend un marqueur détectable, le procédé comprenant en outre l'utilisation du marqueur détectable afin d'identifier, parmi les sondes d'ADN isolées, celles qui s'hybrident au réseau.

3. Procédé selon la revendication 2, dans lequel le marqueur détectable, de préférence la biotine, est à une extrémité 5' du brin.

4. Procédé selon la revendication 1, 2 ou 3, dans lequel chacune des sondes d'hybridation immobilisées comprend une région se liant à une sonde de facteur de transcription comprenant au moins deux copies d'un complément à une partie de la séquence de reconnaissance de facteur de transcription sur une des sondes d'ADN.

5. Procédé pour cribler des modulateurs d'un facteur de transcription, le procédé comprenant :
la formation d'une pluralité d'échantillons de test par la mise en contact d'échantillons de cellules avec différents agents ; et
pour chaque échantillon de test, l'identification, parmi une pluralité de facteurs de transcription activés différents, de ceux qui sont présents par
la mise en contact de l'échantillon de test avec une banque de sondes d'ADN double brin dans des conditions où des complexes sonde d'ADN-facteur de transcription sont formés entre les sondes d'ADN dans la banque et les facteurs de transcription activés présents dans l'échantillon de test, les sondes d'ADN comprenant chacune une séquence de reconnaissance de facteur de transcription connue qui est variable au sein de la banque de sorte que des sondes d'ADN différentes se lient à des facteurs de transcription activés différents, où une sonde d'ADN différente est mise à disposition pour chaque facteur de transcription activé différent dans la pluralité de facteurs de transcription activés,
l'isolement des sondes d'ADN qui se sont liées aux facteurs de transcription activés et ont formé les complexes sonde d'ADN-facteur de transcription, et
la mise en contact des sondes d'ADN isolées avec un réseau de sondes d'hybridation immobilisées dans des conditions appropriées pour permettre une hybridation d'au moins un des brins des sondes d'ADN double brin différentes aux sondes d'hybridation dans le réseau, où une sonde d'hybridation différente est mise à disposition pour chaque sonde d'ADN double brin différente, et où l'identification des sondes d'ADN liées au réseau permet d'identifier, parmi la pluralité de facteurs de transcription activés différents, ceux qui sont présents dans l'échantillon de test ; et
la comparaison des facteurs de transcription activés présents dans les différents échantillons de test.

6. Procédé selon la revendication 5, dans lequel chacune des sondes d'hybridation immobilisées comprend une région se liant à une sonde de facteur de transcription comprenant au moins deux copies d'un complément à une partie de la séquence de reconnaissance de facteur de transcription sur une des sondes d'ADN.

7. Procédé selon la revendication 5 ou la revendication 6, dans lequel un brin de chacune des sondes d'ADN double brin comprend un marqueur détectable, de préférence la biotine, de préférence à une extrémité 5' du brin.

8. Procédé selon l'une quelconque des revendications précédentes, dans lequel la banque comprend des sondes d'ADN double brin comportant des séquences de reconnaissance de facteur de transcription ayant une longueur comprise entre 20 et 40 paires de bases, de préférence une longueur comprise entre 25 et 35 paires de bases.

9. Procédé selon l'une quelconque des revendications précédentes, dans lequel la banque de sondes d'ADN double brin comprend au moins 5, de préférence au moins 10, de préférence au moins 20, de manière davantage préférée au moins 50 séquences de reconnaissance de facteur de transcription différentes.

10. Procédé selon l'une quelconque des revendications précédentes, dans lequel la banque de sondes d'ADN double brin comprend des séquences de reconnaissance de facteur de transcription pour au moins 2, de préférence au moins 5, de manière davantage préférée au moins 10 types de cellules différents.

11. Procédé selon l'une quelconque des revendications précédentes, dans lequel la banque de sondes d'ADN double brin comprend des séquences de reconnaissance de facteur de transcription pour des types de cellules malins, bénins et normaux.

12. Procédé selon l'une quelconque des revendications précédentes, dans lequel l'échantillon biologique est un extrait nucléaire.

13. Procédé selon l'une quelconque des revendications précédentes, dans lequel l'isolement des sondes d'ADN qui se sont liées aux facteurs de transcription activés et ont formé les complexes sonde d'ADN-facteur de transcription comprend la liaison des complexes sonde d'ADN-facteur de transcription à un filtre de nitrocellulose.

14. Procédé selon l'une quelconque des revendications 1 à 12, dans lequel l'isolement des sondes d'ADN qui se sont liées aux facteurs de transcription activés et ont formé les complexes sonde d'ADN-facteur de transcription comprend une séparation entre les complexes sonde d'ADN-facteur de transcription et les sondes d'ADN non liées à des facteurs de transcription activés en utilisant un gel d'agarose.

15. Kit destiné à être utilisé pour identifier, parmi une pluralité de facteurs de transcription activés différents, ceux qui sont présents dans un échantillon biologique en identifiant, parmi les membres d'une banque de sondes d'ADN double brin, ceux qui forment des complexes sonde d'ADN-facteur de transcription avec des facteurs de transcription activés présents dans l'échantillon biologique, le kit comprenant :
la banque de sondes d'ADN double brin, les sondes d'ADN comprenant chacune une séquence de reconnaissance de facteur de transcription connue qui est variable au sein de la banque de sorte que des sondes d'ADN différentes se lient à des facteurs de transcription activés différents, où une sonde d'ADN différente est mise à disposition pour chaque facteur de transcription activé différent dans la pluralité de facteurs de transcription activés ; et
un réseau d'hybridation comprenant :
un substrat ; et
une pluralité de sondes d'hybridation immobilisées sur une surface du substrat de sorte que des sondes d'hybridation différentes soient positionnées dans des régions définies différentes sur la surface, où une sonde d'hybridation différente est mise à disposition pour chaque sonde d'ADN double brin différente, et où chaque sonde d'hybridation différente comprend une région se liant à une sonde de facteur de transcription comprenant au moins deux copies d'un complément à une partie de la séquence de reconnaissance de facteur de transcription sur une des sondes d'ADN.

16. Kit selon la revendication 15, dans lequel le réseau comprend en outre un étalon interne, de préférence un ADN biotinylé.

17. Kit selon la revendication 15 ou 16, dans lequel le réseau comprend en outre un ADN biotinylé qui est positionné sur le réseau de sorte à définir un motif de réseau en grille.

18. Kit selon l'une quelconque des revendications 15 à 17, dans lequel les différentes sondes d'hybridation sur le réseau sont collectivement capables d'immobiliser au moins 5, de préférence au moins 10, de préférence au moins 20, de manière davantage préférée au moins 50 sondes d'ADN différentes au réseau.

19. Kit selon l'une quelconque des revendications 15 à 18, dans lequel les différentes sondes d'hybridation sur le réseau sont collectivement capables d'immobiliser des sondes d'ADN pour au moins 2, de préférence au moins 5, de manière davantage préférée au moins 10 types de cellules différents au réseau.

20. Kit selon l'une quelconque des revendications 15 à 19, dans lequel chaque sonde d'hybridation différente est positionnée dans deux régions différentes ou davantage.

21. Kit selon la revendication 20, dans lequel la concentration de chaque sonde d'hybridation différente est variable dans les deux régions différentes ou davantage.
